# EUROPEAN PATENT APPLICATION

(11) **EP 3 960 730 A1**
(43) Date of publication of application: **02.03.2022**
(21) Application number: 20795185.6
(22) Date of filing: 22.04.2020
(51) Int. Cl.: C07C 303/02, C07C 303/28, C07C 309/75, C07C 309/87

(54) **METHOD FOR PRODUCING ARYLSULFONIC ACID ESTER COMPOUND**

(30) Priority: 26.04.2019 JP 2019085954
(71) Applicant: Nissan Chemical Corporation, Tokyo 103-6119 (JP)
(72) Inventor: KONDO Mitsumasa, Funabashi-shi, Chiba 274-8507 (JP); SHIMADA Junpei, Toyama-shi, Toyama 939-2792 (JP)
(74) Representative: Mewburn Ellis LLP
(86) International application number: PCT/JP2020/017276
(87) International publication number: WO 2020/218316

(57) **Abstract**

Provided is a method for producing an arylsulfonic acid ester compound which comprises: a first step, in which an arylsulfonic acid of formula (1) 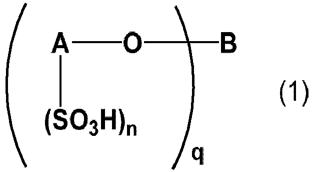 (wherein A is an aromatic group, B is an aromatic group substituted by a halogen atom, n is an integer satisfying 1 ≤ n ≤ 4, and q is an integer satisfying 1 ≤ q) is reacted with a halogenating reagent in the presence of a DMF catalyst to produce a halide of formula (2) 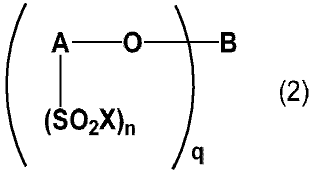 (wherein X is a halogen atom and A, B, n, and q are as defined above),
wherein a solution of the arylsulfonic acid in DME or in diethylene glycol diethyl ether is dropped into the halogenating reagent; and a second step, in which the obtained halide is reacted at 15°C or lower with an alcohol in an organic solvent in the presence of a base of formula (3) 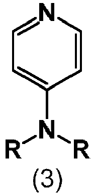 (wherein the R moieties each independently represent an alkyl group optionally containing a heteroatom, but the two R moieties may be bonded to each other to form a ring structure).

## Description

### TECHNICAL FIELD

The present invention relates to a method for producing an arylsulfonic acid ester compound.

### BACKGROUND ART

Organic electroluminescence (hereinafter, referred to as organic EL) elements are expected to be put into practical use in fields such as displays and lighting, and various developments related to materials and element structures have been made for the purpose of achieving low voltage drive, high luminance, long life, and the like.

In this organic EL element, a plurality of functional thin films are used. A hole injection layer, which is one of the functional thin films, is responsible for charge transfer between an anode and a hole transport layer or a light emission layer, and plays an important role in achieving low voltage drive and high luminance of the organic EL element.

In recent years, a charge-transporting varnish including a homogeneous solution in which a low-molecular-weight oligoaniline-based material or an oligothiophene-based material is dissolved in an organic solvent has been found, and it has been reported that a flattening effect of an underlying substrate and excellent organic EL element characteristics can be obtained by inserting a hole injection layer obtained from this varnish into an organic EL element.

The present applicant has already reported that an arylsulfonic acid ester compound is suitable as an electron-accepting substance of these low-molecular-weight oligoaniline-based materials (see Patent Documents 1 and 2).

Meanwhile, in the above Patent Documents 1 and 2, the arylsulfonic acid ester compound is produced by converting a pyridinium salt or a sodium salt of an arylsulfonic acid compound into acid chloride with thionyl chloride in a solvent such as DMF (first step) and then esterifying the resulting compound in a base such as pyridine (second step).

However, regarding the first step, since the methods of Patent Documents 1 and 2 in which a salt is used as a starting material excessively require a step of converting arylsulfonic acid into a salt, it is not suitable for mass production, and thus a method of directly converting arylsulfonic acid into acid chloride is desired. In this method, however, rapid generation of acid gas makes difficult to control the conversion, and there has been a problem in terms of safety. Further, when the scale becomes larger, a large lump is generated during the reaction, having made stirring difficult in some cases. Furthermore, since concentration to dryness is performed to distill off an excessive amount of the thionyl chloride after the reaction, there has been also a problem that a target product cannot be reproducibly obtained, and thus it has been not suitable for mass production.

In addition in the esterification in the second step, if the scale increases, reproducibility is low in terms of reaction time, yield, and the like, and in particular, if the number of esterification sites increases, decomposition of an acid chloride as a raw material or an esterified substance as a product increases. Therefore, it is difficult to obtain a target product in a high yield, and a step such as concentration to dryness is required, and thus there has been room for improvement to efficiently obtain a target product in a high yield.

From such a viewpoint, a new method for producing an arylsulfonic acid ester adaptable to mass production has been desired.

### PRIOR ART DOCUMENTS

### PATENT DOCUMENTS

Patent Document 1: WO 2007/099808 A
Patent Document 2: WO 2017/217457 A

### SUMMARY OF INVENTION

### TECHNICAL PROBLEM

The present invention has been made in view of such circumstances, and an object thereof is to provide a method for producing an arylsulfonic acid ester compound, which is safe, has high reproducibility of reaction time, yield, and the like, and in which a target product can be obtained in a high yield.

### SOLUTION TO PROBLEM

As a result of intensive studies to achieve the above object, the present inventors have found that the above problems can be solved by adding a solution of an arylsulfonic acid compound in 1,2-dimethoxyethane or in diethylene glycol diethyl ether dropwise into a halogenation reagent to produce an arylsulfonic acid halide, and further esterifying this sulfonic acid halide at a low temperature using a specific base, and have completed the present invention.

That is, the present invention provides:
1. a method for producing an arylsulfonic acid ester compound, the method including:
   a first step in which, when an arylsulfonic acid compound is reacted with a halogenation reagent in presence of a catalyst of N,N-dimethylformamide to produce an arylsulfonic acid halide, the arylsulfonic acid compound having formula (1) below: 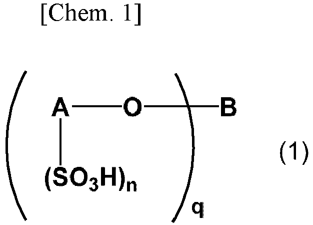 wherein A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
   the arylsulfonic acid halide having formula (2) below: 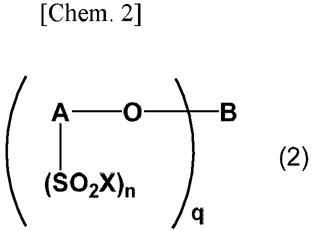 wherein X represents a halogen atom, and A, B, n and q each represent the same meaning as described above,
   a solution of the arylsulfonic acid compound in 1,2-dimethoxyethane or in diethylene glycol diethyl ether is added dropwise into the halogenation reagent to halogenate the sulfo groups and synthesize the arylsulfonic acid halide; and
   a second step of reacting the arylsulfonic acid halide obtained by the first step in an organic solvent and in presence of a base at 15°C or lower with an alcohol compound to synthesize the arylsulfonic acid ester compound, the base having formula (3) below: 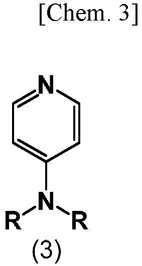 wherein Rs each independently represent a C1-C10 alkyl group optionally including a heteroatom, but two Rs may be bonded to form a ring structure together with a nitrogen atom,
   the alcohol compound having formula (4) below: 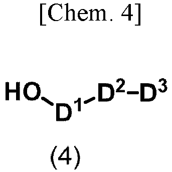 wherein D¹ represents a substituted or unsubstituted divalent hydrocarbon group, D² represents a single bond, O, S, or a substituted or unsubstituted divalent amino group, and D³ represents a substituted or unsubstituted monovalent hydrocarbon group, but D³ may be a hydrogen atom when D² is a single bond,
   the arylsulfonic acid ester compound having formula (5) below: 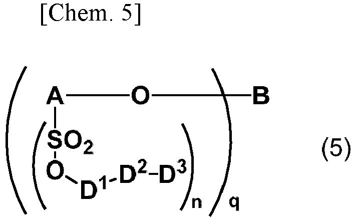 wherein A, B, D¹, D², D³, n and q each represent the same meaning as described above;
2. a method for producing an arylsulfonic acid ester compound, the method including:
   reacting an arylsulfonic acid halide in an organic solvent and in presence of a base at 15°C or lower with an alcohol compound to synthesize the arylsulfonic acid ester compound, the arylsulfonic acid halide having formula (2) below: 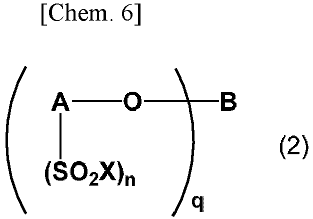 wherein X represents a halogen atom, A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
   the base having formula (3) below: 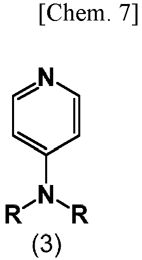 wherein Rs each independently represent a C1-C10 alkyl group optionally including a heteroatom, but two Rs may be bonded to form a ring structure together with a nitrogen atom,
   the alcohol compound having formula (4) below: 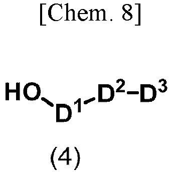 wherein D¹ represents a substituted or unsubstituted divalent hydrocarbon group, D² represents a single bond, O, S, or a substituted or unsubstituted divalent amino group, and D³ represents a substituted or unsubstituted monovalent hydrocarbon group, but D³ may be a hydrogen atom when D² is a single bond,
   the arylsulfonic acid ester compound having formula (5) below: 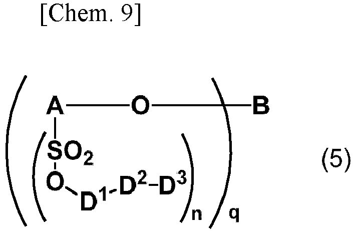 wherein A, B, D¹, D², D³, n and q represent the same meaning as described above;
3. the method for producing an arylsulfonic acid ester compound according to 1 or 2, wherein the base of the formula (3) is N,N-dimethyl-4-aminopyridine or 4-morpholinopyridine;
4. the method for producing an arylsulfonic acid ester compound according to any one of 1 to 3, wherein the reaction between the arylsulfonic acid halide and the alcohol compound is performed at -30 to 10°C;
5. the method for producing an arylsulfonic acid ester compound according to 4, wherein the reaction between the arylsulfonic acid halide and the alcohol compound is performed at -20 to 10°C;
6. the method for producing an arylsulfonic acid ester compound according to any one of 1 to 5, wherein the base of the formula (3) is used in an amount of 1.1 mol or more per 1 mol of sulfonic acid halide groups of the arylsulfonic acid halide;
7. the method for producing an arylsulfonic acid ester compound according to any one of 1 to 6, wherein the B is a C6-C20 q-valent aromatic group in which at least one hydrogen atom of the B is substituted with a fluorine atom;
8. the method for producing an arylsulfonic acid ester compound according to 7, wherein the B is a C6-C20 q-valent aromatic group in which all hydrogen atoms of the B are each substituted with a fluorine atom;
9. the method for producing an arylsulfonic acid ester compound according to any one of 1 to 8, wherein the q is 2;
10. the method for producing an arylsulfonic acid ester compound according to 9, wherein the B is a divalent group having formula (B1) below: 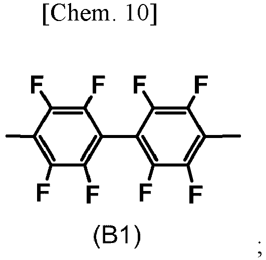
11. the method for producing an arylsulfonic acid ester compound according to any one of 1 to 8, wherein the q is 1;
12. the method for producing an arylsulfonic acid ester compound according to 11, wherein the B is a divalent group of any one having formulas (B2) to (B6) below: 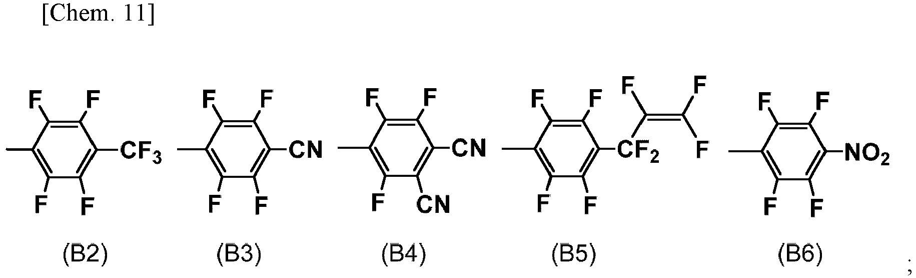
13. the method for producing an arylsulfonic acid ester compound according to any one of 1 to 12, wherein the A is a benzene ring or a naphthalene ring;
14. the method for producing an arylsulfonic acid ester compound according to 1 or any one of 3 to 13, wherein the halogenation reagent is thionyl chloride;
15. the method for producing an arylsulfonic acid ester compound according to 1 or any one of 3 to 14, wherein the first step further includes an operation of adding a poor solvent to a reaction solution that has been obtained to precipitate the arylsulfonic acid halide after synthesizing the arylsulfonic acid halide;
16. a method for producing an arylsulfonic acid halide by reacting an arylsulfonic acid compound with a halogenation reagent in presence of a catalyst of N,N-dimethylformamide, the arylsulfonic acid compound having formula (1) below: 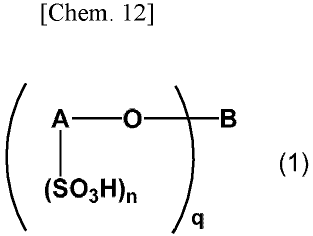 wherein A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
   the arylsulfonic acid halide having formula (2) below: 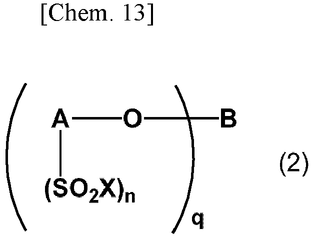 wherein X represents a halogen atom, and A, B, n and q represent the same meaning as described above,
   the method including:
      adding a solution of the arylsulfonic acid compound in 1,2-dimethoxyethane or in diethylene glycol diethyl ether dropwise into the halogenation reagent to halogenate the sulfo groups; and
17. the method for producing an arylsulfonic acid halide according to 16, the method further including:
   an operation of adding a poor solvent to a reaction solution that has been obtained to precipitate the arylsulfonic acid halide after synthesizing the arylsulfonic acid halide.

### ADVANTAGEOUS EFFECTS OF INVENTION

The method for producing an arylsulfonic acid ester compound of the present invention is excellent in safety because gas generation at the time of producing an acid halide can be appropriately controlled, and has not only high reproducibility of reaction time, yield, and the like in an esterification reaction, but also a high reaction rate and good yield of a target product.

Furthermore, the production method of the present invention also has an advantage that steps such as concentration to dryness after the halogenation reaction and after the esterification reaction can be simplified.

The production method of the present invention having such characteristics is useful not only as a synthesis method on a laboratory scale but also as an industrial production method for mass production.

### DESCRIPTION OF EMBODIMENTS

Hereinafter, the present invention is described in more detail.

### (1) First step

As described above, a first step of a method for producing an arylsulfonic acid ester compound according to the present invention is a step in which, when an arylsulfonic acid compound represented by the following formula (1) is reacted with a halogenation reagent in the presence of a catalyst of N,N-dimethylformamide (hereinafter, abbreviated as DMF) to produce an arylsulfonic acid halide represented by the following formula (2), a solution of an arylsulfonic acid compound in 1,2-dimethoxyethane (hereinafter, may be abbreviated as DME) or in diethylene glycol diethyl ether (hereinafter, may be abbreviated as DEGDEE) is added dropwise into the halogenation reagent to halogenate a sulfo group.

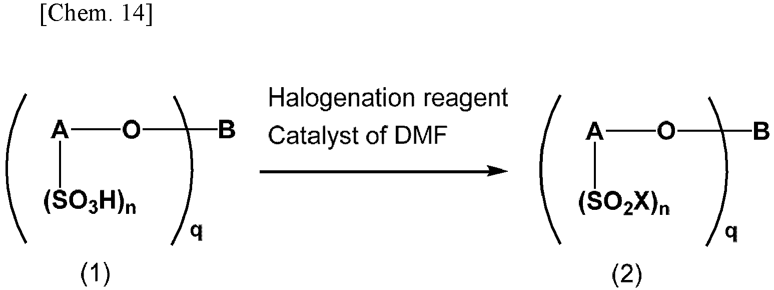

In the respective formulas, A represents a C6-C20 (n+1)-valent aromatic group, B represents a C6-C20 q-valent aromatic group substituted with a halogen atom, n represents the number of sulfo groups bonded to A and is an integer satisfying 1 ≤ n ≤ 4, q represents the number of bonds between B and an oxygen atom and is an integer satisfying 1 ≤ q, and X represents a halogen atom.

Examples of the C6-C20 aromatic group of A include a benzene ring, a naphthalene ring, an anthracene ring, and a biphenyl ring, but preferred are a naphthalene ring and an anthracene ring, and more preferred is a naphthalene ring. Note that A may be substituted with a substituent other than a sulfo group such as a halogen atom, a cyano group, or a nitro group.

Examples of the C6-C20 aromatic group of B include a benzene ring, a naphthalene ring, an anthracene ring, and a biphenyl ring. Preferred are a benzene ring, a naphthalene ring, and a biphenyl ring, and more preferred is a biphenyl ring.

Examples of the halogen atom of B include a fluorine atom, a chlorine atom, a bromine atom, and an iodine atom, and preferred is a fluorine atom. Note that B may be substituted with a substituent other than a halogen atom, such as a cyano group, a nitro group, or a perfluoroallyl group.

B is a C6-C20 aromatic group in which at least one, preferably two or more, and still more preferably all hydrogen atoms thereof are each substituted with a fluorine atom (the C6-C20 aromatic group having a perfluoro structure). Among them, preferred are a 4-trifluoromethyl-2,3,5,6-tetrafluorophenyl group, a 4-cyano-2,3,5,6-tetrafluorophenyl group, a 4-nitro-2,3,5,6-tetrafluorophenyl group, a 3,4-dicyano-2,5,6-trifluorophenyl group, a 4-perfluoroallyl-2,3,5,6-tetrafluorophenyl group, and a perfluorobiphenyldiyl group, and more preferred are a perfluorobiphenyl-4,4'-diyl group and a 4-trifluoromethyl-2,3,5,6-tetrafluorophenyl group.

In addition, n represents the number of sulfo groups bonded to A, and is an integer satisfying 1 ≤ n ≤ 4, but is preferably an integer of 2 to 4, more preferably 2 or 3, and still more preferably 2.

q represents the number of bonds between B and an oxygen atom and is an integer satisfying 1 ≤ q, but is preferably an integer of 1 to 4, more preferably 1 or 2, and still more preferably 2.

Examples of the halogen atom of X include those exemplified in the above B, but preferred are a chlorine atom and a bromine atom, and more preferred is a chlorine atom.

Examples of the A-(SO₃H)ₙ group suitable in the present invention include, but are not limited to, groups represented by the following formulas (A1) and (A2). Among them, preferred is one represented by formula (A1).

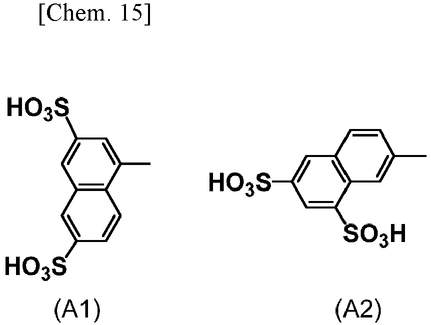

In addition, examples of B suitable in the present invention include, but are not limited to, those represented by the following formulas (B1) to (B6). Among them, suitable is one represented by formula (B1).

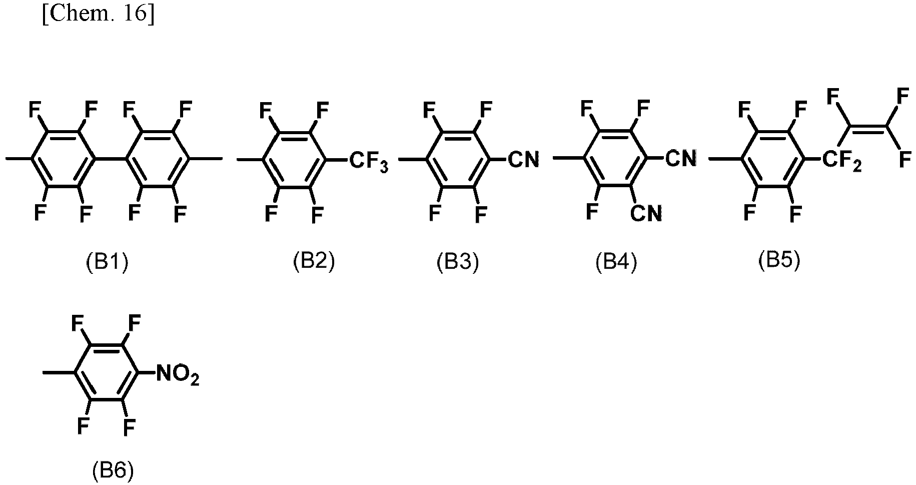

Specific examples of the arylsulfonic acid compound represented by the formula (1) as a raw material for the first step of the production method of the present invention include, but are not limited to, those represented by the following formulas.

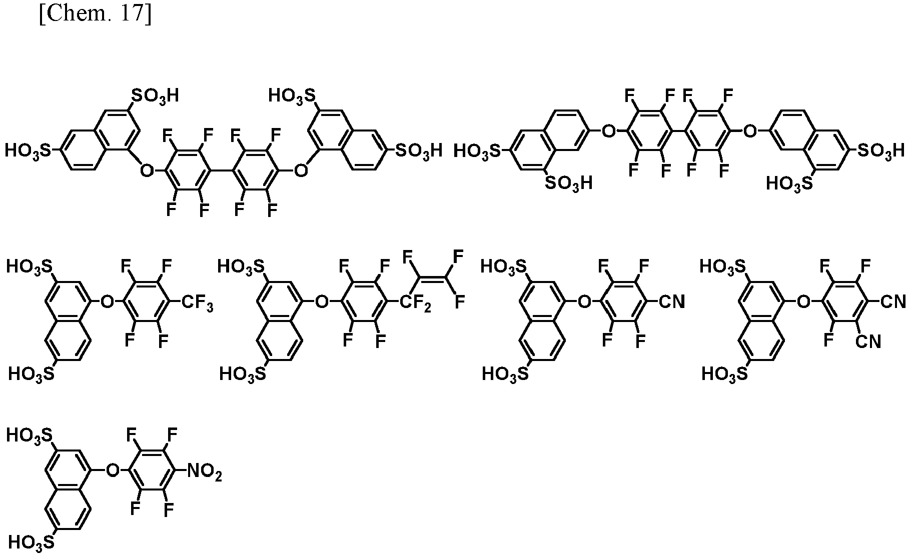

In the first step, in the DME first step used for preparing the solution of the arylsulfonic acid compound in DME, the amount of DME or DEGDEE used for preparing the solution of the arylsulfonic acid compound in DME or in DEGDEE is not particularly limited as long as the arylsulfonic acid compound is dissolved therein. However, the amount of DME or DEGDEE is, per a value of 1 for arylsulfonic acid as a weight ratio, preferably 0.1 to 10, more preferably 1 to 7, and still more preferably 1 to 3, in consideration of the balance between the solubility and the dropping amount of the arylsulfonic acid compound.

The halogenation reagent can be appropriately selected from conventionally known reagents and used, and examples thereof include thionyl chloride, oxalyl chloride, phosphoryl chloride, sulfuryl chloride, phosphorus trichloride, and phosphorus pentachloride, but preferred is thionyl chloride in consideration of reactivity, safety, workability, price, and the like.

The amount of the halogenation reagent used is not limited as long as it is equal to or more than the theoretical amount by which all sulfo groups of the arylsulfonic acid compound are halogenated, but too small amount thereof may delay the progress of the reaction or cause impurities derived from DMF used as a catalyst to remain. Therefore, from the viewpoint of the reaction rate, the yield and purity of the sulfonic acid compound after crystallization, workability, reproducibility, and cost, typically, the halogenation reagent is usually used at about 1 to 10 times by weight per the arylsulfonic acid compound, and the lower limit value thereof is preferably 1.5 times by weight, and the upper limit thereof is preferably 5 times by weight and more preferably 3.5 times by weight.

In the present invention, the solution of the arylsulfonic acid compound in DME or in DEGDEE may be added dropwise to the halogenation reagent itself, or the solution of DME or the solution of DEGDEE may be added dropwise to a mixture of the halogenation reagent and the organic solvent. In this case, the organic solvent to be used is not particularly limited as long as it is an organic solvent that does not affect the reaction, but DME or DEGDEE used for dissolving the arylsulfonic acid compound is preferred.

In the first step of the production method of the present invention, a catalyst such as DMF is added for the purpose of accelerating the reaction and improving the conversion rate. The amount of DMF used as the catalyst is not particularly limited, but too small amount thereof may delay the progress of the reaction, whereas too large amount thereof may cause impurities derived from DMF to be generated. Therefore, in consideration of the balance between these conditions, the amount is preferably 0.025 mol or more, more preferably 0.025 to 0.25 mol, and still more preferably 0.075 to 0.125 mol per 1 mol of the sulfo group of the arylsulfonic acid. The molar equivalent (hereinafter, denoted as eq) with respect to 1 mol of the arylsulfonic acid can be appropriately set in accordance with the number of sulfo groups of the arylsulfonic acid. For example, in a case where the arylsulfonic acid has four sulfo groups, the molar equivalent is preferably 0.1 eq or more, more preferably 0.1 to 1 eq, and still more preferably 0.3 to 0.5 eq.

The catalyst such as DMF may be added into the solution of the arylsulfonic acid in DME or in DEGDEE to be added dropwise, added into the halogenation reagent, or added into both of the solution of DME and the solution of DEGDEE, but it is preferred to add the catalyst into the halogenated reagent from the viewpoint of the reaction rate, ease of gas generation control, simplification of operation, and the like.

The reaction temperature is not particularly limited as long as the reaction proceeds, but is preferably about 30 to 85°C, more preferably 50 to 85°C, and still more preferably 60 to 80°C in consideration of rapidly advancing the reaction and appropriately adjusting the generation of acid gas.

Note that it is preferable to heat the halogenation reagent to the above temperature range in advance upon dropwise addition of the solution of the arylsulfonic acid compound in DME or in DEGDEE.

The reaction time is appropriately set in consideration of the type and amount of the catalyst to be used, the reaction temperature, and the like, and is usually about 1 to 48 hours.

After the end of the reaction, a poor solvent is added to precipitate an arylsulfonic acid halide, which is then filtered off and washed, dried and the like, whereby a target product can be obtained. In the present invention, by adding a poor solvent to the reaction solution to precipitate an arylsulfonic acid halide, the halogenation reagent remaining in the reaction solution can be easily and reliably removed, and the target product can be obtained with good reproducibility, as compared with a method of isolating the arylsulfonic acid halide by concentrating the reaction solution.

The poor solvent is not particularly limited as long as the target arylsulfonic acid halide is precipitated and has no adverse effect such as promotion of decomposition of the arylsulfonic acid halide. Examples thereof include aliphatic hydrocarbons (pentane, n-hexane, n-heptane, n-octane, n-decane, decalin, and the like), aromatic hydrocarbons (benzene, nitrobenzene, toluene, o-xylene, m-xylene, p-xylene, mesitylene, and the like), halogenated aromatic hydrocarbons (chlorobenzene, bromobenzene, o-dichlorobenzene, m-dichlorobenzene, p-dichlorobenzene, and the like), ethers (diethyl ether, diisopropyl ether, t-butyl methyl ether, tetrahydrofuran, 1,4-dioxane, 1,2-dimethoxyethane, 1,2-diethoxyethane, diethylene glycol diethyl ether, and the like), ketones (acetone, methyl ethyl ketone, methyl isobutyl ketone, di-n-butyl ketone, cyclohexanone, and the like), nitriles (acetonitrile, propionitrile, butyronitrile, and the like), and these solvents may be used singly, or two or more of these may be used in combination. Among them, preferred are aliphatic hydrocarbons (pentane, n-hexane, n-heptane, n-octane, n-decane, decalin, and the like), aromatic hydrocarbons (benzene, nitrobenzene, toluene, o-xylene, m-xylene, p-xylene, mesitylene, and the like), ethers (diethyl ether, diisopropyl ether, t-butyl methyl ether, tetrahydrofuran, 1,4-dioxane, 1,2-dimethoxyethane, 1,2-diethoxyethane, diethylene glycol diethyl ether, and the like), ketones (acetone, methyl ethyl ketone, methyl isobutyl ketone, di-n-butyl ketone, cyclohexanone, and the like), and nitriles (acetonitrile, propionitrile, butyronitrile, and the like), and more preferred are toluene, n-heptane, and acetonitrile.

### (2) Second step

A second step of the method for producing an arylsulfonic acid ester compound according to the present invention is a step of synthesizing an arylsulfonic acid ester compound represented by the following formula (5) by reacting the arylsulfonic acid halide obtained in the first step as described above with an alcohol compound represented by the following formula (4) at 15°C or less in an organic solvent and in the presence of a base represented by the following formula (3).

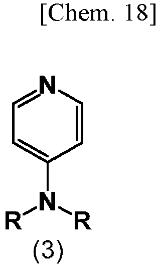

In formula (3), Rs each independently represent a C1-C10 alkyl group optionally including a heteroatom, but two Rs may be bonded to form a ring structure together with a nitrogen atom.

The C1-C10 alkyl group of R may be any of a linear, branched, or cyclic group, and examples thereof include linear or branched C1-C10 alkyl groups such as methyl, ethyl, n-propyl, isopropyl, n-butyl, isobutyl, s-butyl, t-butyl, n-pentyl, n-hexyl, n-heptyl, n-octyl, n-nonyl, and n-decyl groups; and cyclic C3-C20 alkyl groups such as cyclopropyl, cyclobutyl, cyclopentyl, cyclohexyl, cycloheptyl, cyclooctyl, cyclononyl, cyclodecyl, bicyclobutyl, and bicyclopentyl groups.

In addition, examples of the heteroatom include an oxygen atom, a sulfur atom, and a nitrogen atom.

Among them, R is preferably a C1-C5 alkyl group optionally including a heteroatom, or a C2-C5 alkylene group formed by bonding Rs to each other, preferably methyl, ethyl, tetramethylene, pentamethylene, or a 3-oxapentamethylene group, more preferably two Rs each being a methyl group, two Rs each being an ethyl group, or a 3-oxapentamethylene group in which two Rs bonded to each other.

Specific examples of the base represented by the formula (3) include N,N-dimethyl-4-aminopyridine (hereinafter, abbreviated as DMAP), N,N-diethyl-4-aminopyridine, and 4-morpholinopyridine (hereinafter, abbreviated as 4MP), and DMAP is optimal in consideration of increasing the reaction rate and increasing the yield of the target product by esterifying all sulfonic acid halide groups.

The amount of the base used and represented by formula (3) is not particularly limited, but too small amount thereof may delay the progress of the reaction, whereas too large amount thereof may advance the decomposition of the arylsulfonic acid halogen compound and the target product. Thus, in consideration of increasing the reaction rate and increasing the yield of the target product by esterifying all sulfonic acid halide groups, the amount is preferably 1.1 mol or more, more preferably 1.1 to 1.5 mol, and still more preferably 1.1 to 1.3 mol, per 1 mol of the sulfonic acid halide groups of the arylsulfonic acid halide. The molar equivalent (eq) per 1 mol of the arylsulfonic acid halide can be appropriately set in accordance with the number of sulfonic acid halide group of the arylsulfonic acid halide, and for example, in a case where the arylsulfonic acid halide has four sulfonic acid halide groups, the molar equivalent is preferably 4.4 eq or more, more preferably 4.4 to 6.0 eq, and still more preferably 4.4 to 5.2 eq.

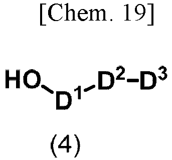

In formula (4), D¹ represents a substituted or unsubstituted divalent hydrocarbon group, D² represents a single bond, O, S, or a substituted or unsubstituted divalent amino group, and D³ represents a substituted or unsubstituted monovalent hydrocarbon group, but D³ may be a hydrogen atom in a case where D² is a single bond.

Examples of the substituted or unsubstituted divalent hydrocarbon group of D¹ include substituted or unsubstituted C1-C5 alkylene groups, C1-C2 alkyleneoxy-C1-C2 alkylene groups, C1-C2 alkylenethio-C1-C2 alkylene groups, C1-C2 alkylene carbonyl-C1-C2 alkylene groups, and groups in which some or all of hydrogen atoms of these groups are further substituted with a hydroxyl group, an amino group, a silanol group, a thiol group, a carboxyl group, a sulfonic acid ester group, a phosphoric acid group, a phosphoric acid ester group, an ester group, a thioester group, an amide group, a nitro group, a monovalent hydrocarbon group, an organooxy group, an organoamino group, an organosilyl group, an organothio group, an acyl group, a sulfone group, a halogen atom, and the like. In the present invention, preferred is a C1-C5 alkylene group. Examples of the C1-C5 alkylene group include methylene, ethylene, propylene, trimethylene, tetramethylene, and pentamethylene groups, and preferred are methylene, ethylene, propylene, and trimethylene groups.

D² is a single bond, O, S, or a substituted or unsubstituted divalent amino group, but O is preferred in the present invention. Here, examples of the divalent substituted amino group include -N(CH₃)-, -N(C₂H₅)-, and -N(C₃H₇)-.

D³ represents a substituted or unsubstituted monovalent hydrocarbon group, but may be a hydrogen atom in a case where D² is a single bond. Examples of the substituted or unsubstituted monovalent hydrocarbon group include alkyl groups such as methyl, ethyl, n-propyl, i-propyl, n-butyl, i-butyl, t-butyl, n-hexyl, n-octyl, 2-ethylhexyl, and decyl groups; cycloalkyl groups such as cyclopentyl and cyclohexyl groups; bicycloalkyl groups such as a bicyclohexyl group; alkenyl groups such as vinyl, 1-propenyl, 2-propenyl, isopropenyl, 1-methyl-2-propenyl, 1-butenyl, 2-butenyl, 3-butenyl, and hexenyl groups; aromatic ring groups (aryl groups) such as phenyl, xylyl, tolyl, biphenyl, and naphthyl groups; aralkyl groups such as benzyl, phenylethyl, and phenylcyclohexyl groups; and groups in which some or all of the hydrogen atoms of these groups are further substituted with the above-described substituents. In the present invention, preferred are methyl, ethyl, n-propyl, n-butyl and phenyl groups.

In addition, the alcohol compound represented by formula (4) is preferably an alcohol compound represented by the following formula (4') in which the above D¹, D² and D³ are taken together with each other to form a structure represented by the following formula (D').

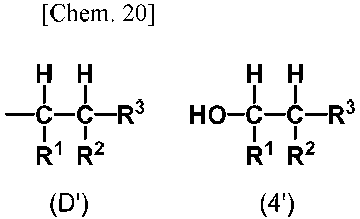

In formula (D') and formula (4'), R¹ and R² each independently represent a hydrogen atom, a linear or branched monovalent aliphatic hydrocarbon group, and R³ represents a linear or branched monovalent aliphatic hydrocarbon group or an alkoxy group. Provided that the total number of carbon atoms of R¹, R², and R³ is 2 or more. The total number of carbon atoms of R¹, R², and R³ is not particularly limited, but is preferably 20 or less, and more preferably 10 or less.

The linear or branched monovalent aliphatic hydrocarbon group is not particularly limited, and examples thereof include C1-C18 alkyl groups such as methyl, ethyl, n-propyl, i-propyl, n-butyl, i-butyl, t-butyl, n-hexyl, n-octyl, 2-ethylhexyl, and decyl groups; and C2-C18 alkenyl groups such as vinyl, 1-propenyl, 2-propenyl, isopropenyl, 1-methyl-2-propenyl, 1-butenyl, 2-butenyl, 3-butenyl, and hexenyl groups.

The alkoxy group is preferably a C1-C10 alkoxy group, and specific examples thereof include methoxy, ethoxy, n-propoxy, i-propoxy, n-butoxy, s-butoxy, t-butoxy, n-pentoxy, and phenoxy groups.

R¹ is more preferably a methyl group. R² is preferably a hydrogen atom. R³ is preferably an alkoxy group, more preferably a methoxy, ethoxy, n-propoxy, n-butoxy, or phenoxy group, and still more preferably an ethoxy, n-butoxy, or phenoxy group.

Specific examples of the alcohol compound represented by formula (4) include, but are not limited to, alcohols such as methanol, ethanol, n-propanol, i-propanol, n-butanol, i-butanol, sec-butanol, t-butanol, n-pentanol, n-hexanol, cyclohexanol, n-heptanol, cycloheptanol, n-octanol, 2-ethyl-1-hexanol, n-nonanol, 3-nonanol, and 2-butyl-1-octanol; and glycol monoethers such as propylene glycol monoethyl ether, propylene glycol monopropyl ether, propylene glycol monobutyl ether, propylene glycol monophenyl ether, ethylene glycol monobutyl ether, and ethylene glycol monohexyl ether.

Among them, preferred are 2-ethyl-1-hexanol, 2-butyl-1-octanol, 1-octanol, propylene glycol monoethyl ether, propylene glycol monopropyl ether, propylene glycol monobutyl ether, propylene glycol monophenyl ether, ethylene glycol monobutyl ether, and ethylene glycol monohexyl ether.

The amount of the alcohol compound used is not particularly limited, but is, per a value of 1 for the arylsulfonic acid halide as a weight ratio, preferably 2 to 10, more preferably 3 to 7, and still more preferably 3.5 to 6 from the viewpoint of the reaction rate and the solubility of a base.

In addition, the amount of the alcohol compound in a solution or slurry of the arylsulfonic acid halide and the alcohol compound is, per a value of 1 for the arylsulfonic acid halide as a weight ratio, preferably 2 to 10, and more preferably 3 to 6. 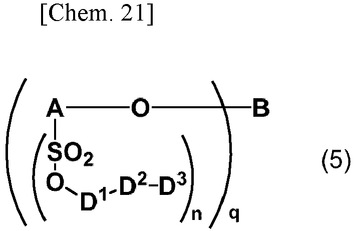 (In the formula, A, B, D¹, D², D³, n and q represent the same meaning as described above.)

In addition, preferred embodiments of the arylsulfonic acid ester compound represented by the formula (5) include, but are not limited to, an arylsulfonic acid ester compound represented by the following formula (5'). 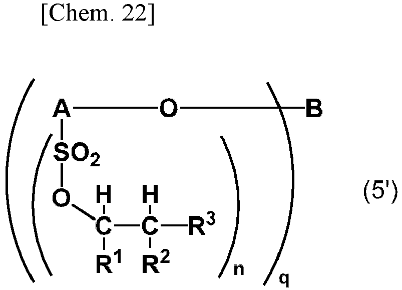 (In the formula, A, B, R¹, R², R³, n and q represent the same meaning as described above.)

The operation of the reaction in the second step is not particularly limited as long as the arylsulfonic acid halide represented by formula (2) is reacted with the alcohol compound represented by formula (4) at 15°C or lower in the presence of the base represented by formula (3) in an organic solvent to suitably give the arylsulfonic acid ester compound represented by formula (5). A mixed solution of: one or both of the alcohol compound represented by formula (4) and the organic solvent; and the base represented by formula (3), may be added dropwise into a mixed solution or slurry of: the arylsulfonic acid halide represented by formula (2); and one or both of the alcohol compound represented by formula (4) and the organic solvent. However, in consideration of the stability of the arylsulfonic acid halide represented by formula (2) and the target product, the reaction rate, the solubility of the base, workability, ease of temperature management during the reaction, and the like, it is desirable to dropwise add: a mixed solution of the alcohol compound represented by formula (4), the organic solvent, and the base represented by formula (3); or a mixed solution of the organic solvent and the base represented by formula (3), into a solution or slurry of the arylsulfonic acid halide represented by formula (2) and the alcohol compound represented by formula (4). In this case, when both the composition to be added dropwise (for example, the composition in the dropping funnel) and the composition to be received dropwise (for example, the composition in the reaction flask) contain the alcohol compound represented by formula (4), from the viewpoint of obtaining a target ester form in a high yield, it is necessary to use only the alcohol compound corresponding to the ester form.

The organic solvent is not particularly limited as long as it does not adversely affect the reaction. Specific examples thereof include aliphatic hydrocarbons (pentane, n-hexane, n-octane, n-decane, decalin, and the like), halogenated aliphatic hydrocarbons (chloroform, dichloromethane, dichloroethane, carbon tetrachloride, and the like), aromatic hydrocarbons (benzene, nitrobenzene, toluene, o-xylene, m-xylene, p-xylene, mesitylene, and the like), halogenated aromatic hydrocarbons (chlorobenzene, bromobenzene, o-dichlorobenzene, m-dichlorobenzene, p-dichlorobenzene, and the like), ethers (diethyl ether, diisopropyl ether, t-butyl methyl ether, tetrahydrofuran, 1,4-dioxane, 1,2-dimethoxyethane, 1,2-diethoxyethane, diethylene glycol diethyl ether, and the like), ketones (acetone, methyl ethyl ketone, methyl isobutyl ketone, di-n-butyl ketone, cyclohexanone, and the like), amides (N,N-dimethylformamide, N,N-dimethylacetamide, and the like), lactams and lactones (N-methyl-2-pyrrolidone, γ-butyrolactone, and the like), ureas (N,N-dimethylimidazolidinone, tetramethylurea, and the like), sulfoxides (dimethylsulfoxide, sulfolane, and the like), and nitriles (acetonitrile, propionitrile, butyronitrile, and the like).

In addition, as the organic solvent that can be used in the reaction of the second step, in addition to the above-described organic solvent, the alcohol compound represented by formula (4) can also be used. That is, in the present invention, the alcohol compound represented by formula (4) can function as both the raw material compound and the organic solvent.

Note that the solvents may be used singly, or two or more of these may be used in combination.

Among them, preferred are ethers (diethyl ether, diisopropyl ether, t-butyl methyl ether, tetrahydrofuran, 1,4-dioxane, 1,2-dimethoxyethane, 1,2-diethoxyethane, diethylene glycol diethyl ether, and the like), ketones (acetone, methyl ethyl ketone, methyl isobutyl ketone, di-n-butyl ketone, cyclohexanone, and the like), nitriles (acetonitrile, propionitrile, butyronitrile, and the like), and lactams (N-methyl-2-pyrrolidone and the like), and more preferred are acetonitrile, propionitrile, tetrahydrofuran, and N-methyl-2-pyrrolidone (hereinafter, abbreviated as NMP).

The amount of the organic solvent used is not particularly limited as long as it does not affect the reaction and is an amount that dissolves the base in a case where a mixed solution containing the base represented by formula (3) is added dropwise. However, too large amount thereof may delay the reaction, and too small amount thereof may prevent completion of the reaction. Therefore, per a value of 1 for the arylsulfonic acid halide as a weight ratio, the amount of the organic solvent used is usually 1 to 10, preferably 2 to 10, more preferably 2 to 7, still more preferably 2 to 6, and still further preferably 2 to 4.

In addition, in a case where an organic solvent in an amount in which the base represented by the formula (3) cannot be dissolved is used, per a value of 1 for the arylsulfonic acid halide as a weight ratio, an alcohol compound corresponding to the target ester form of a value of 1 to 2 may be added thereto, whereby the base can be dissolved.

Examples of the A-(SO₃-D¹-D²-D³)ₙ group suitable in the present invention include groups represented by the following formulas (A3) and (A4), but are not limited thereto. Among them, preferred is one represented by formula (A3). 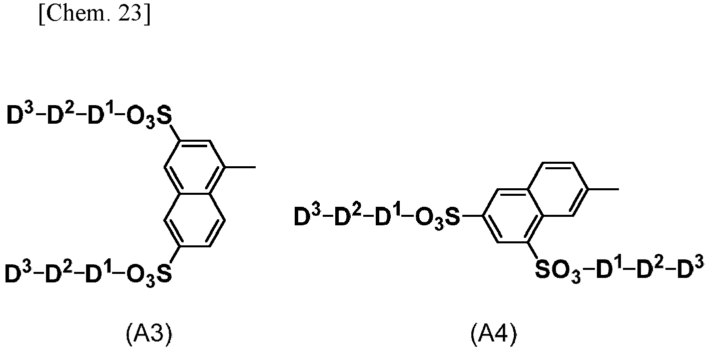 (In the formula, D¹, D² and D³ represent the same meaning as described above.)

In addition, more preferred embodiments of the A-(SO₃-D¹-D²-D³)ₙ group include, but are not limited to, groups represented by the following formulas (A3') and (A4'). Among them, preferred is one represented by formula (A3'). 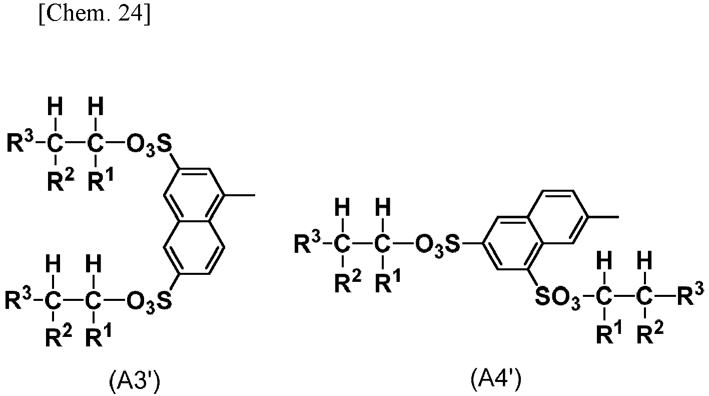 (In the formula, R¹, R² and R³ represent the same meaning as described above.)

In addition, B suitable for the second step of the present invention is the same as B suitable for the first step.

Preferred embodiments of the arylsulfonic acid ester compound obtained by the production method of the present invention include, but are not limited to, those listed below. 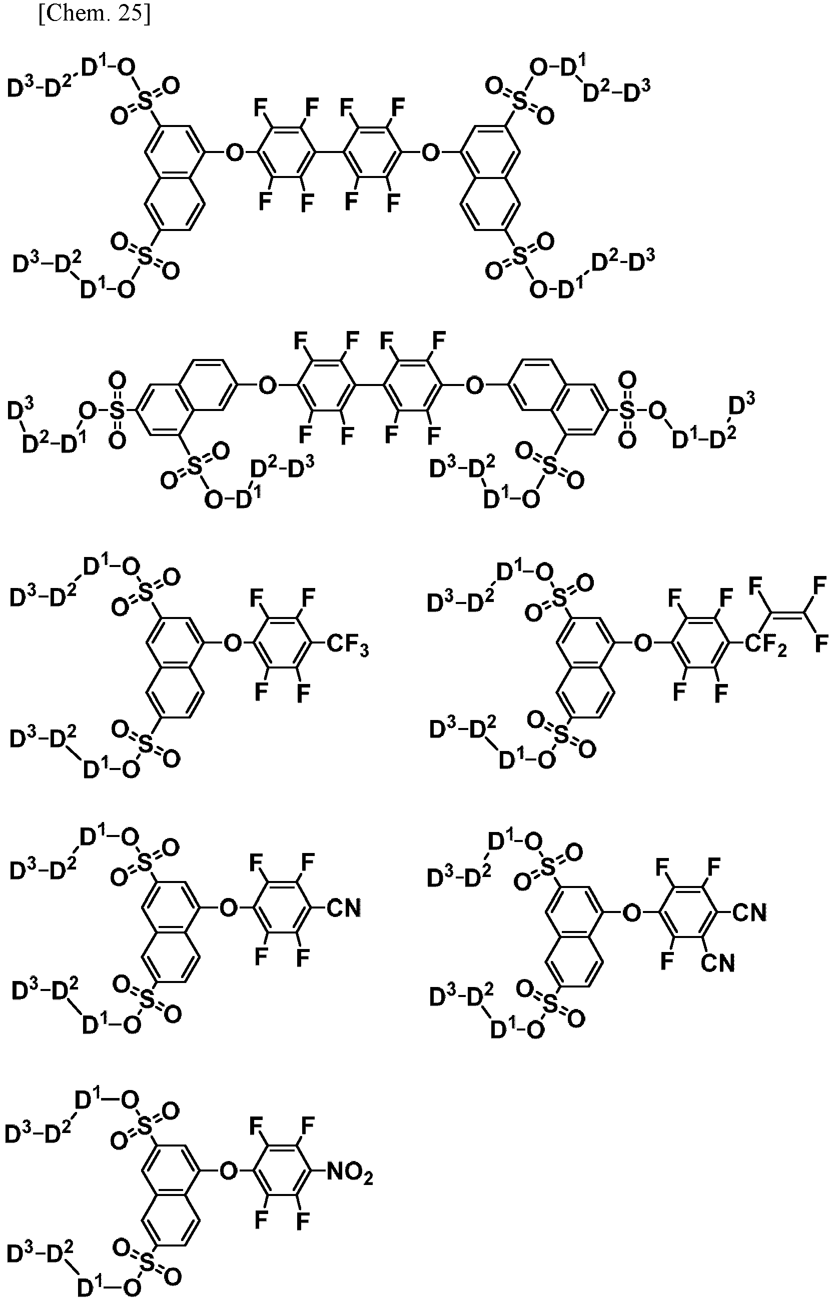 (In the formula, D¹, D² and D³ represent the same meaning as described above.)

In addition, more preferred embodiments of the arylsulfonic acid ester compound obtained by the production method of the present invention include, but are not limited to, those listed below. 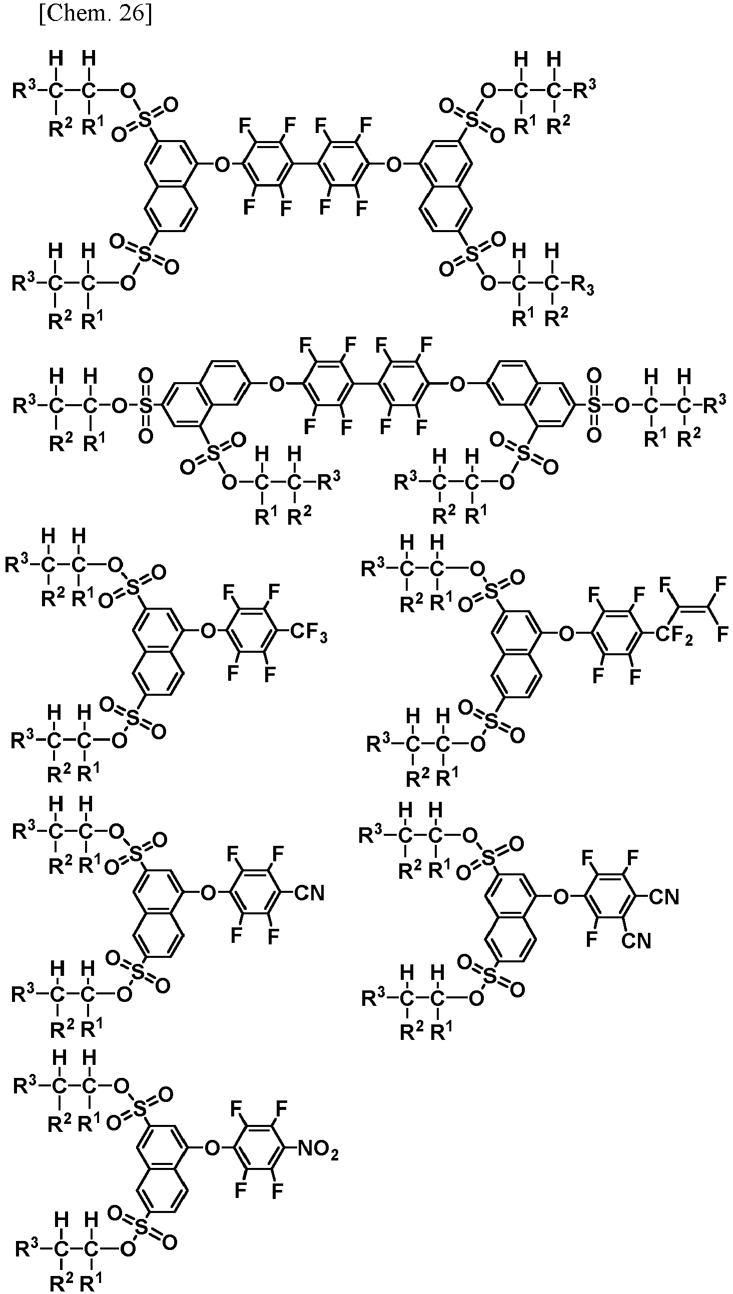 (In the formula, R¹, R² and R³ represent the same meaning as described above.)

The water content in the reaction system of the second step is preferably 10 mol% or less, more preferably 8 mol% or less, still more preferably 7 mol% or less from the viewpoint of favorably advancing the reaction. The lower limit value is not particularly limited, but is usually about 1 mol%.

Therefore, as the reagent used in the second step, a dehydrated reagent is preferably used, and in particular as the organic solvent, one having a moisture content of less than 50 ppm is preferably used.

Preferred embodiments of the second step include, but is not limited to, a method of adding a mixed solution of an alcohol compound to be used for esterification, a base represented by formula (3), and an organic solvent containing NMP or acetonitrile into a liquid obtained by dissolving or suspending an arylsulfonic acid halide in an alcohol compound to be used for esterification by a dropping method.

Note that in this case, the addition amount of NMP or acetonitrile is, per a value of 1 for the arylsulfonic acid halide as a weight ratio, usually 1 to 10, preferably 2 to 10, more preferably 2 to 7, still more preferably 2 to 6, and still further preferably 2 to 4.

In the present invention, the reaction temperature in the second step is 15°C or lower from the viewpoint of making the reaction time appropriate and suppressing side reactions, but is preferably -30 to 12°C, more preferably -25 to 10°C, still more preferably -20 to 10°C, still further preferably -15 to 0°C, and particularly preferably -15 to -5°C.

The reaction time is appropriately determined in consideration of the amount of the base to be used, the reaction temperature, and the like, and is usually about 1 to 48 hours. If the reaction solution is left to stand for a long time after the end (completion) of the reaction, side reactant may increase, and therefore it is preferable to perform post-treatment quickly after the end of the reaction. Whether or not the reaction has ended can be confirmed by, for example, reaction tracking using liquid chromatography, but the present invention is not limited to this method.

After the end of the reaction, the reaction solution is filtered, and the filtered material is washed with a solvent such as ethyl acetate, then washed with a hydrochloric acid aqueous solution, an ammonium chloride aqueous solution, or the like, whereby a target product can be obtained. Note that the washing step is also preferably performed at a low temperature from the viewpoint of suppressing the decomposition of the target product. The temperature is typically 10°C or lower, and the lower limit value thereof is not particularly limited unless the solution is frozen, but is usually about -10°C.

Specific examples of the arylsulfonic acid ester compound suitably obtained by the production method of the present invention include, but are not limited to, those listed below.

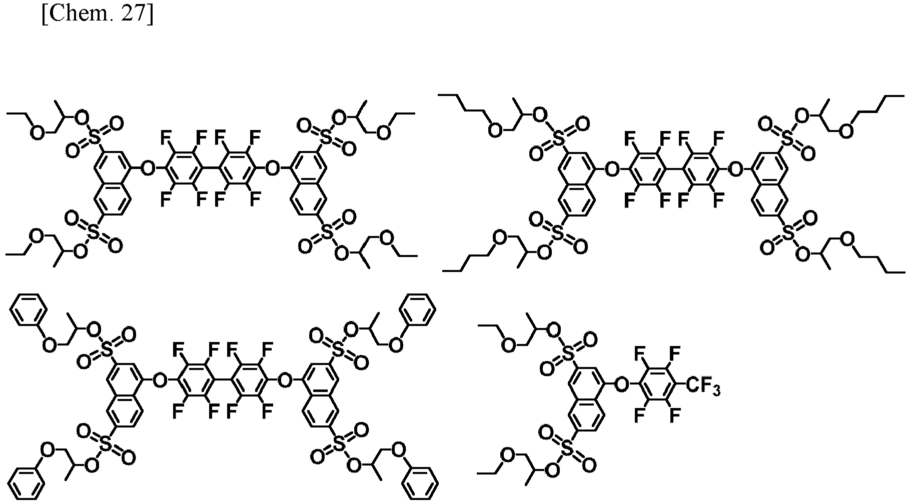

Note that the esterification by the method of the second step using the arylsulfonic acid halide obtained in the first step has been mentioned in the above description, but the method of the second step in the present invention can be applied to the esterification of the arylsulfonic acid halide produced by a method other than the method of the first step, and the raw material thereof is not limited to the halide obtained in the first step.

### EXAMPLES

Hereinafter, the present invention is described more specifically with reference to Examples and Comparative Examples, but the present invention is not limited to the following Examples. Note that the apparatuses used are described below. In addition, in Examples and Comparative Examples, times by weight means a weight ratio per 1 part by weight of arylsulfonic acid X or X-2, or arylsulfonic acid X-Cl or X-Cl-2, all of which are described later and are a starting material of respective steps.

- PGEE:: Propylene glycol monoethyl ether
- DMF:: N,N-Dimethylformamide
- NMP: N-Methyl-2-pyrrolidone
- MeCN:: Acetonitrile
- DME:: 1,2-Dimethoxyethane
- DMAc:: N,N-Dimethylacetamide
- THF:: Tetrahydrofuran
- DMAP:: N,N-Dimethylaminopyridine
- 4MP: 4-Morpholinopyridine
- NMI:: N-Methylimidazole
- Et₃N:: Triethylamine
- Py:: Pyridine
- NEM:: N-Ethylmorpholine
- DEGDEE:: Diethylene glycol diethyl ether
- SOCl₂:: Thionyl chloride

**[¹H-NMR]**

| | |
|---|---|
| Apparatus: | Fourier transform superconducting nuclear magnetic resonance apparatus (FT-NMR) "INOVA-400" 400 MHz, manufactured by Varian, Inc. |
| Solvent: | DMSO-d6, CDCl₃ |
| Internal standard substance: | Tetramethylsilane (TMS) |

**[LC-MS]**

| | |
|---|---|
| Apparatus: | Q-Exactive (manufactured by Thermo) |
| Mode: | APCI-APPI |
| Column: | XBridge C18, 2.1 mm × 150 mm, 5 µm |
| Flow rate: | 0.22 mL/min |
| Temperature: | 45°C |

### [HPLC]

### <Preparation of HPLC analytical solution>

In HPLC analysis of the reaction solution obtained in the synthesis step of the later-described arylsulfonic acid X-PGEE and arylsulfonic acid X-Cl as a raw material thereof, an unreacted acid chloride that is amidated using CH₃CN was used for analysis.

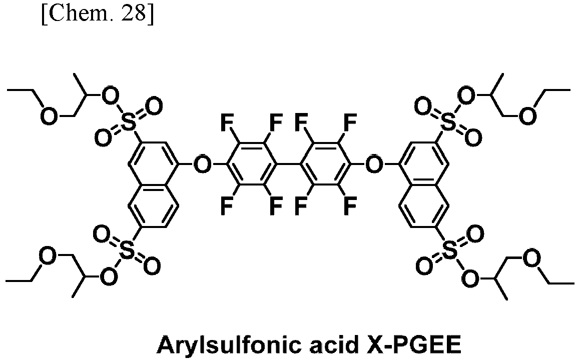

### <Analysis conditions>

| | |
|---|---|
| Apparatus: | Liquid chromatography "Prominence-i", manufactured by SHIMADZU CORPORATION |
| Column: | Poroshell 120 EC-C18 2.7 µm, 3.0 × 50 mm (Agilent) |
| Oven: | 40°C |
| Detection wavelength: | 254 nm |
| Flow rate: | 0.8 mL/min |
| Eluent: | Gradient |

| (A) Water : | (B) acetonitrile |
|---|---|
| Gradient condition; | 0-5 minutes (B40A60 → B100), 5-15 minutes (B100), 15-20 minutes (B100 → B40A60), 20-30 minutes (B40A60) |
| Sample injection amount: | 2 µL |
| Data collection time: | 20 minutes |

The relationship between the structure of the compound represented by the following formula (P) and the retention time of HPLC is listed in Table 1 and 2. Here, regarding R₁ to R₄ of the following formula (P), "Cl" in the table indicates that unreacted Cl is n-Bu₂N-converted, and "A" indicates a group represented by the following formula (Q). 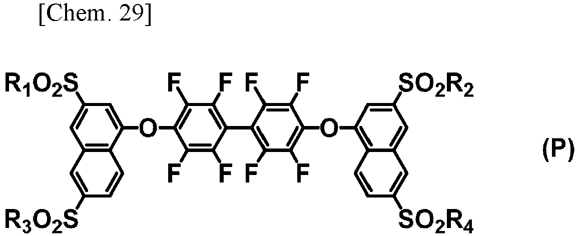 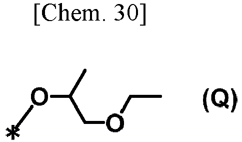 (In the formula, * represents a bond.)

**[Table 1]**

| | | R₁ | R₂ | R₃ | R₄ | Retention time (min) |
|---|---|---|---|---|---|---|
| Raw material | Arylsulfonic acid X-Cl | Cl | Cl | Cl | Cl | 12.3 |
| Intermediate | Monosubstituted ester | A | Cl | Cl | Cl | 9.9 to 11.5 |
| | | Cl | A | Cl | Cl | |
| | | Cl | Cl | A | Cl | |
| | | Cl | Cl | Cl | A | |
| | Disubstituted ester | A | A | Cl | Cl | |
| | | Cl | Cl | A | A | |
| | | A | Cl | A | Cl | |
| | | A | Cl | Cl | A | |
| | | Cl | A | Cl | A | |
| | | Cl | A | A | Cl | |
| | Trisubstituted ester | A | A | A | Cl | |
| | | A | A | Cl | A | |
| | | A | Cl | A | A | |
| | | Cl | A | A | A | |
| Target product | Arylsulfonic acid X-PGEE | A | A | A | A | 9.3 |

**[Table 2]**

| | | R₁ | R₂ | R₃ | R₄ | Retention time (min) |
|---|---|---|---|---|---|---|
| Decomposition product | Trisubstituted product | OH | A | A | A | 5.7,6.3 |
| | | A | OH | A | A | |
| | | A | A | OH | A | |
| | | A | A | A | OH | |
| | Decomposition product (including decomposition intermediates) | OH | Cl | Cl | Cl | 7.9, 8.1 |
| | | Cl | OH | Cl | Cl | |
| | | Cl | Cl | OH | Cl | |
| | | Cl | Cl | Cl | OH | |
| | | OH | OH | Cl or A | Cl or A | 1 to 2.7 |
| | | Cl or A | Cl or A | OH | OH | |
| | | OH | Cl or A | OH | Cl or A | |
| | | OH | Cl or A | Cl or A | OH | |
| | | Cl or A | OH | Cl or A | OH | |
| | | Cl or A | OH | OH | Cl or A | |
| | | OH | OH | OH | Cl or A | |
| | | OH | OH | Cl or A | OH | |
| | | OH | Cl or A | OH | OH | |
| | | Cl or A | OH | OH | OH | |
| Arylsulfonic acid X | | OH | OH | OH | OH | 1 |

In Examples and Comparative Examples described later, the peak area was calculated for each peak appearing in the chromatogram obtained by HPLC measurement of the reaction solution, and the ratio of the product contained in the reaction solution was determined based on the relationship with the retention time listed in Tables 1 and 2.

### [Example 1-1] Synthesis of arylsulfonic acid X-Cl

A sulfonyl halide compound (arylsulfonic acid X-Cl) was synthesized according to the following procedure.

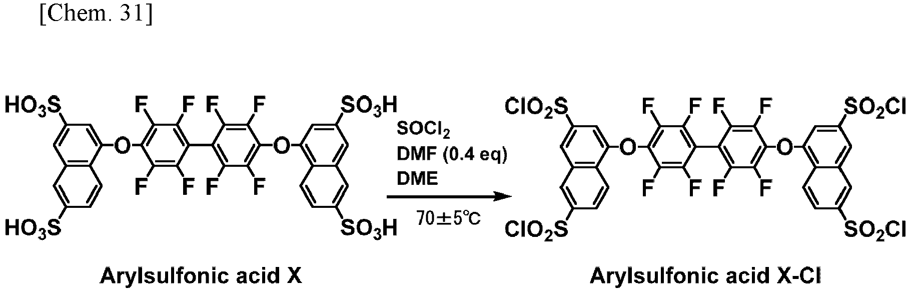

Under a nitrogen atmosphere, 245 g (3 times by weight) of thionyl chloride and 2.6 g (0.4 eq) of DMF were added to a 1L four-necked flask, and the resulting mixture was stirred at 73°C for 1 hour. At 40°C, 30 g of arylsulfonic acid X was added to 232 g (2.9 times by weight) of DME and dissolved. Further, 30 g of arylsulfonic acid X and 20 g of arylsulfonic acid X were added portionwise and dissolved, and then transferred to a dropping funnel and washed with 8 g (0.1 times by weight) of 1,2-dimethoxyethane. This solution was added dropwise to a mixed solution of thionyl chloride and DMF at 73°C over 4 hours, and the resulting mixture was stirred for 19 hours. At this time, acid gas is generated until the end of the reaction. Thus, the acid gas was released from the upper portion of the reflux tube to the outside of the system through the sodium hydroxide aqueous solution. In addition at this time, no lump was generated, and stirring was good. Furthermore, the arylsulfonic acid X-Cl was precipitated during the dropwise addition. The reaction solution after 19 hours was subjected to HPLC analysis, and the result indicated that the reaction liquid contained an arylsulfonic acid X-Cl (98.0%) and a trisubstituted product (0.3%).

After confirming the termination of the reaction, 240 g (3 times by weight) of n-heptane was added dropwise at 73°C to 60°C over 1 hour. After the end of the dropwise addition, the resulting mixture was cooled to 25°C over 2 hours and stirred for 2 hours. The precipitated arylsulfonic acid X-Cl was filtered under nitrogen pressure, and the filtered material was washed three times with 160 g (2 times by weight) of a solution of n-heptane and 1,2-dimethoxyethane with a weight ratio of n-heptane-to-1,2-dimethoxyethane of 4:1. Thereafter, the filtered material was washed twice with 80 g (1 time by weight) of n-heptane. The obtained crystals were dried under reduced pressure at 60°C to give 80.6 g of arylsulfonic acid X-Cl (yield: 93.1%, LC area: 98.0%). The measurement results of ¹H-NMR are given below.

| | |
|---|---|
| ¹H-NMR (400MHz, CDCl₃): | δ 7.49 (s, 2H), 8.39, 8.42 (d, J = 0.03 Hz, 2H), 8.64 (s, 2H), 8.78, 8.81 (d, J = 0.03 Hz, 2H), 8.87, 8.88 (d, J = 0.01 Hz, 2H) |

In Example 1-1, it was confirmed that a solution of arylsulfonic acid X in DME was added dropwise to a solution of thionyl chloride in DMF, thereby making it possible to adjust the amount of acid gas generated without failure of stirring, and to produce arylsulfonic acid X-Cl safely and efficiently.

### [Example 1-2] Synthesis of arylsulfonic acid X-PGEE

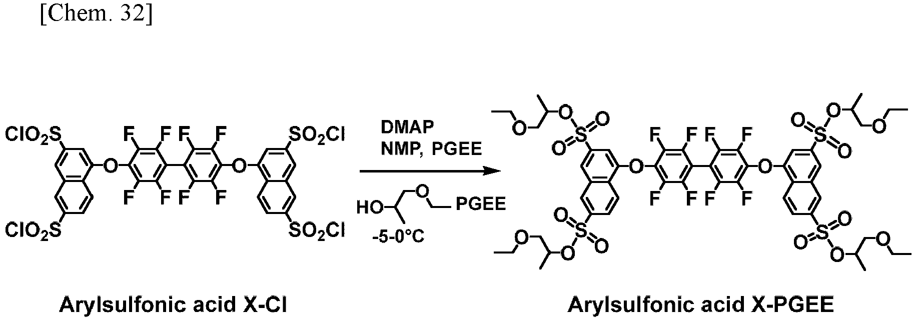

Under a nitrogen atmosphere, 60.0 g of arylsulfonic acid X-Cl and 180 g (3 times by weight) of dehydrated PGEE were added to a 500 mL four-necked flask, and the resulting mixture was cooled to -3°C. After cooling, a mixed solution of 29.0 g (3.9 eq) of DMAP, 128.6 g (2.1 times by weight) of dehydrated NMP, and 51.4 g (0.9 times by weight) of dehydrated PGEE was added dropwise thereto at -3°C to -0.6°C over 45 minutes. Thereafter, a mixed solution of DMAP (4.8 g, 0.6 eq), 21.4 g (0.4 times by weight) of dehydrated NMP, and 8.6 g (0.1 times by weight) of dehydrated PGEE was added dropwise thereto at -1.7°C to -1.1°C over 3 hours, and the resulting mixture was stirred at -4.8°C for 21 hours. The reaction solution after 21 hours was subjected to HPLC analysis, and the result indicated that the reaction solution contained arylsulfonic acid X-PGEE (96.2%), a trisubstituted product (3.3%), and raw material and intermediate (0.02%). Thereafter, the reaction solution was filtered, and the filtered material was washed twice with 120 g (2 times by weight) of ethyl acetate cooled to 4°C. The obtained filtrate was heated to 5.5°C, 360 g (6 times by weight) of ethyl acetate cooled to 4°C was added, and the resulting mixture was stirred. At 5°C to 8°C, 600 g (10 times by weight) of a 15 wt% ammonium chloride aqueous solution cooled to 5°C was added dropwise, and the mixture was continuously stirred at 5°C for 10 minutes, and then allowed to stand for 3 minutes. After removing the aqueous layer, stirring was resumed, and 600 g (10 times by weight) of a 15 wt% ammonium chloride aqueous solution cooled to 5°C was again added dropwise at 5°C to 5.5°C, and the mixture was continuously stirred at 5°C for 10 minutes, and then allowed to stand for 5 minutes. After removing the aqueous layer, stirring was resumed, and 300 g (5 times by weight) of a 15 wt% ammonium chloride aqueous solution cooled to 5°C was added dropwise at 5°C, and stirring was continued at 5°C for 10 minutes, and then the mixture was allowed to stand for 7 minutes. After removing the aqueous layer, 270 g (4.5 times by weight) of diisopropyl ether was added. A 95 mm KIRIYAMA ROHTO (KIRIYAMA funnel) was filled with 240 g (4 times by weight) of neutral silica gel 60N suspended in 720 g (12 times by weight) of a solution of ethyl acetate and diisopropyl ether with a weight ratio of ethyl acetate-to-diisopropyl ether of 2:1, and the separated solution was filtered. After the filtration, the mixture was further filtered with 1,880 g (31 times by weight) of a solution of ethyl acetate and diisopropyl ether with a weight ratio of ethyl acetate-to-diisopropyl ether of 2:1, and the silica gel on a filter was washed. The obtained solution was concentrated under reduced pressure to 462 g (7.7 times by weight) at 25°C, and ethyl acetate (82 g, 1.3 times by weight) was added to adjust the total amount to 540 g (9 times by weight). This solution was cooled to 3°C, and isopropanol (1,500 g, 25 times by weight) was added dropwise at 3°C to 5°C over 1 hour with stirring, then the resulting mixture was stirred at 3°C for 23 hours. After confirming precipitation of arylsulfonic acid X-PGEE, this solution was concentrated under reduced pressure to 900 g (15 times by weight) at 25°C, and isopropanol (1,200 g, 20 times by weight) was added thereto. The solution was concentrated under reduced pressure again to 900 g (15 times by weight) at 25°C, and 1,200 g (20 times by weight) of isopropanol was added thereto. The precipitate was filtered, and the filtered material was washed twice with 120 g (2 times by weight) of diisopropyl ether. The obtained crystals were dried under reduced pressure at 25°C and 5 hPa for 12 hours and at 40°C and 5 hPa for 5 hours to give 72.2 g of arylsulfonic acid X-PGEE (yield: 94.2%, LC area: 99.4%). The measurement results of ¹H-NMR and LC/MS are given below.

| | |
|---|---|
| ¹H-NMR (400MHz, CDCl₃): | δ 0.92-0.97 (m, 12H), 1.34 and 1.40 (a pair of d, J = 6.5 Hz, 12H), 3,32-3,52 (m, 16H), 4.80-4.87 (m, 4H), 7.37 (s, 2H), 8.22 (d, J = 8.5 Hz, 2H), 8.45 (s, 2H), 8.61 (d, J = 8.5 Hz, 2H), 8.69 (s, 2H) |
| LC/MS (ESI⁺) m/z; | 1264 [M+NH₄]⁺ |

### [Example 2]

Under a nitrogen atmosphere, 600 mg (3 times by weight) of dehydrated PGEE was added to 200 mg of arylsulfonic acid X-Cl, and a mixed solution of 113 mg (4.5 eq) of DMAP, 500 mg (2.5 times by weight) of dehydrated NMP, and 200 mg (1 time by weight) of dehydrated PGEE was added dropwise thereto at -10°C over about 1 minute, then the resulting mixture was stirred for 23 hours. At that time, a part of the reaction solution was sampled at the time point of the reaction time described in the table and analyzed by HPLC.

### [Example 3]

Synthesis of arylsulfonic acid X-PGEE and analysis by HPLC were performed in the same operation as in Example 2 except that the base was changed to 4MP.

### [Comparative Example 1]

Under a nitrogen atmosphere, 1.66 g (8.3 times by weight) of dehydrated chloroform and 720 mg (3.6 times by weight) of dehydrated pyridine were added dropwise to 200 mg of arylsulfonic acid X-Cl at 0°C over 5 minutes, and 260 mg (1.3 times by weight) of dehydrated PGEE was added dropwise thereto over 3 minutes. Thereafter, the temperature was raised to 25°C, and the resulting mixture was stirred for 23 hours. At that time, a part of the reaction solution was sampled at the time point of the reaction time described in the table and analyzed by HPLC.

### [Comparative Example 2]

Under a nitrogen atmosphere, 1.66 g (8.3 times by weight) of dehydrated chloroform was added to 200 mg of arylsulfonic acid X-Cl at -10°C. At -10°C, 720 mg (3.6 times by weight) of dehydrated pyridine was added dropwise over 1 minute, and 260 mg (1.3 times by weight) of dehydrated PGEE was added dropwise thereto over 3 minutes, then the resulting mixture was stirred for 23 hours. At that time, a part of the reaction solution was sampled at the time point of the reaction time described in the table and analyzed by HPLC.

### [Comparative Example 3]

Under a nitrogen atmosphere, 720 mg (3.6 times by weight) of dehydrated pyridine was added dropwise to 200 mg of arylsulfonic acid X-Cl at -10°C over 2 minutes, and 260 mg (1.3 times by weight) of dehydrated PGEE was added dropwise thereto over 3 minutes, then the resulting mixture was stirred for 23 hours. At that time, a part of the reaction solution was sampled at the time point of the reaction time described in the table and analyzed by HPLC.

### [Comparative Example 4]

Under a nitrogen atmosphere, 720 mg (3.6 times by weight) of dehydrated pyridine was added dropwise to 200 mg of arylsulfonic acid X-Cl at -10°C over 2 minutes, and 800 mg (4 times by weight) of dehydrated PGEE was added dropwise thereto over 3 minutes, then the resulting mixture was stirred for 23 hours. At that time, a part of the reaction solution was sampled at the time point of the reaction time described in the table and analyzed by HPLC.

### [Comparative Example 5]

Under a nitrogen atmosphere, 200 mg (1 time by weight) of dehydrated pyridine was added dropwise to 200 mg of arylsulfonic acid X-Cl at -10°C over 1 minute, and 260 mg (1.3 times by weight) of dehydrated PGEE was added dropwise thereto over 3 minutes, then the resulting mixture was stirred for 23 hours. At that time, a part of the reaction solution was sampled at the time point of the reaction time described in the table and analyzed by HPLC.

### [Comparative Example 6]

Under a nitrogen atmosphere, 800 mg (4 times by weight) of dehydrated PGEE was added to 200 mg of arylsulfonic acid X-Cl, and 700 mg (3.5 times by weight) of dehydrated pyridine was added dropwise thereto at -10°C over 1 minute, then the resulting mixture was stirred for 4 hours. At that time, a part of the reaction solution was sampled at the time point of the reaction time described in the table and analyzed by HPLC.

### [Comparative Example 7]

Under a nitrogen atmosphere, 800 mg (4 times by weight) of dehydrated PGEE was added to 200 mg of arylsulfonic acid X-Cl, 700 mg (43.6 eq) of dehydrated pyridine was added dropwise thereto at 0°C over 1 minute, then the resulting mixture was stirred for 23 hours. At that time, a part of the reaction solution was sampled at the time point of the reaction time described in the table and analyzed by HPLC.

### [Comparative Example 8]

Under a nitrogen atmosphere, 30 g (3 times by weight) of dehydrated PGEE was added to 10g of arylsulfonic acid X-Cl, and a mixed solution of 5.63 g (4.5 eq) of DMAP and 35 g (3.5 times by weight) of dehydrated PGEE was added dropwise thereto at 1°C to 4.5°C over 1 hour, then the resulting mixture was stirred at 4.5°C for 25 hours. At that time, a part of the reaction solution was sampled at the time point of the reaction time described in the table and analyzed by HPLC.

### [Comparative Example 9]

Under a nitrogen atmosphere, 600 mg (3 times by weight) of dehydrated PGEE was added to 200 mg of arylsulfonic acid X-Cl, and a mixed solution of 113 mg (4.5 eq) of DMAP and 700 mg (3.5 times by weight) of dehydrated PGEE was added dropwise thereto at -10°C over 1 minute, and the resulting mixture was stirred for 42 hours. At that time, a part of the reaction solution was sampled at the time point of the reaction time described in the table and analyzed by HPLC.

### [Comparative Example 10]

Under a nitrogen atmosphere, 84g (8.4 times by weight) of dehydrated PGEE was cooled to 4°C. At 4°C, 2 g of arylsulfonic acid X-Cl was added, and the resulting mixture was stirred for 5 minutes. Then, 0.68 g of a 48 wt% sodium hydroxide aqueous solution was added, and the resulting mixture was stirred for 5 minutes. This operation was repeated five times at 2°C to 4°C, then 0.34g of a 48 wt% sodium hydroxide aqueous solution was added, and the mixture was stirred for 42 hours. At that time, a part of the reaction solution was sampled at the time point of the reaction time described in the table and analyzed by HPLC. Thereafter, the precipitate was filtered, and the filtered material was washed twice with 20 g (2 times by weight) of water. The obtained crystals were dried under reduced pressure at 25°C and 5 hPa for 7 hours to give 0.78 g of arylsulfonic acid X-PGEE (yield: 6%, LC area: 87.5%).

The results of Examples 2 and 3 and Comparative Examples 1 to 10 are listed in Table 3.

**[Table 3]**

| | Temp. [°C] | Solvent for arylsulfonic acid X-Cl [times by weight] | Solution added dropwise | | Reaction time [hr] | Peak area [%] | | |
|---|---|---|---|---|---|---|---|---|
| | | | Base [eq] | Solvent [times by weight] | | Arylsulfonic acid X-PGEE | Trisubstituted product | Raw material + intermediate |
| Comparative Example 1 | 0→25 | CHCl₃ [8.3] Py [3.6] *1 | - | PGEE [1.3] | 2 | 59 | 41 | 0 |
| | | | | | 4 | 55 | 45 | 0 |
| | | | | | 23 | 29 | 71 | 0 |
| Comparative Example 2 | -10 | | | | 2 | 22 | 23 | 52 |
| | | | | | 4 | 50 | 49 | 1 |
| | | | | | 23 | 48 | 52 | 0 |
| Comparative Example 3 | | Py [3.6] *1 | | | 2 | 78 | 21 | 0 |
| | | | | | 23 | 71 | 29 | 0 |
| Comparative Example 4 | | | | PGEE [4] | 4 | 67 | 32 | 1 |
| | | | | | 23 | 67 | 33 | 0 |
| Comparative Example 5 | | Py [1] *2 | | PGEE [1.3] | 4 | 56 | 35 | 8 |
| | | | | | 23 | 60 | 40 | 0 |
| Comparative Example 6 | | PGEE [4] | Py [43.6 eq] *3 | - | 4 | 61 | 38 | 1 |
| Comparative Example 7 | 0 | | | | 4 | 82 | 18 | 0 |
| | | | | | 23 | 77 | 22 | 0 |
| Comparative Example 8 | 5 | PGEE [3] | DMAP [4.5] | PGEE [3.5] | 4 | 21 | 1 | 76 |
| | | | | | 25 | 64 | 3 | 31 |
| Comparative Example 9 | -10 | | | | 23 | 38 | 2 | 58 |
| | | | | | 42 | 55 | 4 | 39 |
| Example 2 | -10 | PGEE [3] | DMAP [4.5] | NMP [2.5] PGEE [1] | 4 | 94 | 5 | 0 |
| | | | | | 6 | 93 | 5 | 0 |
| | | | | | 23 | 94 | 5 | 0 |
| Example 3 | | | 4MP [4.5] | | 4 | 92 | 4 | 3 |
| | | | | | 6 | 94 | 4 | 1 |
| | | | | | 23 | 95 | 5 | 0 |
| Comparative Example 10 | 2-4 | PGEE [8] | 48 wt% NaOH aq [4.4] | - | 42 | 32 | 35 | 14 |

| | | | | | | | | |
|---|---|---|---|---|---|---|---|---|
| *1 In Comparative Examples 1 to 4, 3.6 times by weight of Py corresponds to 45 eq as a base. *2 In Comparative Example 5, 1 time by weight of Py corresponds to 12 eq as a base. *3 In Comparative Examples 6 and 7, 43.6 eq of Py corresponds to 3.5 times by weight as a solvent. | | | | | | | | |

### [Comparative Examples 2-1 to 2-4]

Each arylsulfonic acid X-PGEE was synthesized in the same operation as in Example 2 except that a base was changed to the respective compounds listed in Table 4. At that time, a part of the reaction solution was sampled at the time point of the reaction time described in the table and analyzed by HPLC.

The results of Comparative Examples 2-1 to 2-4 are listed in Table 4. Note that the results of Examples 2 and 3 are also listed in Table 4.

**[Table 4]**

| | Temp. [°C] | Solvent for arylsulfonic acid X-Cl [times by weight] | Solution added dropwise | | Reaction time [hr] | Peak area [%] | | |
|---|---|---|---|---|---|---|---|---|
| | | | Base [eq] | Solvent [times by weight] | | Arylsulfonic acid X-PGEE | Trisubstituted product | Raw material + intermediate |
| Example 2 | -10 | PGEE [3] | DMAP [4.5] | NMP [2.5] PGEE [1] | 23 | 94 | 5 | 0 |
| Example 3 | | | 4MP [4.5] | | 23 | 95 | 5 | 0 |
| Comparative Example 2-1 | | | NMI [4.5] | | 6 | 84 | 14 | 2 |
| | | | | | 23 | 85 | 15 | 0 |
| Comp. Example 2-2 | | | Et₃N [4.5] | | 23 | 53 | 20 | 16 |
| Comp. Example 2-3 | | | Py [4.5] | | 22 | 1 | 7 | 20 |
| Comp. Example 2-4 | | | NEM [4.5] | | 22 | 0 | 6 | 54 |

### [Examples 4 and 5]

Each arylsulfonic acid X-PGEE was synthesized in the same operation as in Example 2 except that the amount of the base used was changed to the respective amounts listed in Table 5. At that time, a part of the reaction solution was sampled at the time point of the reaction time described in the table and analyzed by HPLC.

The results of Examples 4 and 5 are listed in Table 5. Note that the results of Example 2 are also listed in Table 5.

**[Table 5]**

| | Temp. [°C] | Solvent for arylsulfonic acid X-Cl [times by weight] | Solution added dropwise | | Reaction time [hr] | Peak area [%] | | |
|---|---|---|---|---|---|---|---|---|
| | | | Base [eq] | Solvent [times by weight] | | Arylsulfonic acid X-PGEE | Trisubstituted product | Raw material + intermediate |
| Example 4 | -10 | PGEE [3] | DMAP [4.6] | NMP [2.5] PGEE [1] | 4 | 93 | 6 | 0 |
| | | | | | 23 | 93 | 6 | 0 |
| Example 2 | | | DMAP [4.5] | | 4 | 94 | 5 | 0 |
| | | | | | 23 | 94 | 5 | 0 |
| Example 5 | | | DMAP [4.4] | | 4 | 93 | 5 | 0 |
| | | | | | 23 | 93 | 6 | 0 |

### [Examples 6 to 13]

Each arylsulfonic acid X-PGEE was synthesized in the same operation as in Example 2 except that the condition for the reaction temperature and/or the conditions for the solvent were changed to the respective conditions listed in Table 6. At that time, a part of the reaction solution was sampled at the time point of the reaction time described in the table and analyzed by HPLC.

The results of Examples 6 to 13 are listed in Table 6. Note that the results of Example 2 are also listed in Table 6.

**[Table 6]**

| | Temp. [°C] | Solvent for arylsulfonic acid X-Cl [times by weight] | Solution added dropwise | | Reaction time [hr] | LC area [%] | | |
|---|---|---|---|---|---|---|---|---|
| | | | Base [eq] | Solvent [times by weight] | | Arylsulfonic acid X-PGEE | Trisubstituted product | Raw material + intermediate |
| Example 6 | -20 | PGEE [3] | DMAP [4.5] | NMP [2.5] PGEE [1] | 6 | 92 | 5 | 2 |
| | | | | | 23 | 94 | 5 | 0 |
| Example 2 | -10 | | | | 6 | 93 | 5 | 0 |
| | | | | | 23 | 94 | 5 | 0 |
| Example 7 | 0 | | | | 6 | 93 | 6 | 0 |
| | | | | | 23 | 92 | 6 | 0 |
| Example 8 | 5 | | | | 4 | 93 | 5 | 0 |
| | | | | | 23 | 91 | 7 | 0 |
| Example 9 | 10 | | | | 4 | 92 | 6 | 0 |
| | | | | | 23 | 88 | 10 | 0 |
| Example 10 | 15 | | | | 4 | 92 | 6 | 0 |
| | | | | | 23 | 85 | 14 | 0 |
| Example 11 | -10 | PGEE [4] | | MeCN [3.5] | 6 | 95 | 4 | 0 |
| | | | | | 23 | 95 | 4 | 0 |
| Example 12 | 0 | | | | 6 | 96 | 3 | 0 |
| | | | | | 23 | 96 | 4 | 0 |
| Example 13 | 10 | | | | 6 | 94 | 5 | 0 |
| | | | | | 23 | 90 | 10 | 0 |

### [Examples 14 to 40]

Synthesis of each arylsulfonic acid X-PGEE and analysis by HPLC were performed in the same operation as in Example 2 except that the conditions for the solvent were changed to the respective conditions listed in Table 7. The results are listed in Table 7. Note that the results of Example 2 are also listed in Table 7.

**[Table 7]**

| | Temp. [°C] | Solvent for arylsulfonic acid X-Cl [times by weight] | Solution added dropwise | | Reaction time [hr] | Peak area [%] | | |
|---|---|---|---|---|---|---|---|---|
| | | | Base [eq] | Solvent [times by weight] | | Arylsulfonic acid X-PGEE | Trisubstituted product | Raw material + intermediate |
| Example 14 | | PGEE [6] | | NMP [3.5] | 6 | 93 | 5 | 2 |
| | | | | | 23 | 94 | 5 | 0 |
| Example 15 | | PGEE [5.5] | | | 6 | 95 | 4 | 0 |
| | | | | | 23 | 95 | 4 | 0 |
| Example 16 | | PGEE [5] | | | 6 | 94 | 4 | 0 |
| | | | | | 23 | 95 | 4 | 0 |
| Example 17 | | PGEE [4.5] | | | 6 | 95 | 4 | 0 |
| | | | | | 23 | 95 | 4 | 0 |
| Example 18 | | PGEE [4] | | | 6 | 94 | 5 | 0 |
| | | | | | 23 | 94 | 5 | 0 |
| Example 19 | | PGEE [3.5] | | | 6 | 93 | 6 | 0 |
| | | | | | 23 | 93 | 6 | 0 |
| Example 20 | | PGEE [3] | | | 6 | 92 | 6 | 0 |
| | | | | | 23 | 92 | 6 | 0 |
| Example 2 | | | | NMP [2.5] PGEE [1] | 4 | 94 | 5 | 0 |
| | | | | | 23 | 94 | 5 | 0 |
| Example 21 | | | | NMP [3.5] PGEE [1] | 4 | 94 | 4 | 1 |
| | | | | | 23 | 95 | 4 | 0 |
| Example 22 | | PGEE [4] | | | 6 | 94 | 5 | 0 |
| | | | | | 23 | 94 | 6 | 0 |
| Example 23 | | PGEE [5] | | | 6 | 93 | 6 | 1 |
| | | | | | 23 | 93 | 6 | 0 |
| Example 24 | | PGEE [5] | | MeCN [3.5] | 6 | 95 | 4 | 0 |
| | | | | | 23 | 95 | 4 | 0 |
| Example 25 | | PGEE [4] | | | 6 | 95 | 4 | 0 |
| | | | | | 23 | 95 | 4 | 0 |
| Example 26 | | PGEE [3.5] | | | 6 | 95 | 4 | 0 |
| | -10 | | DMAP [4.5] | | 23 | 95 | 4 | 0 |
| Example 27 | | PGEE [3] | | | 6 | 94 | 5 | 0 |
| | | | | | 23 | 94 | 5 | 0 |
| Example 28 | | PGEE [2.5] | | | 6 | 93 | 6 | 0 |
| | | | | | 23 | 93 | 6 | 0 |
| Example 29 | | PGEE [2.5] MeCN [0.5] | | | 6 | 93 | 5 | 0 |
| | | | | | 23 | 93 | 6 | 0 |
| Example 30 | | PGEE [2] MeCN [1] | | | 6 | 91 | 8 | 0 |
| | | | | | 23 | 91 | 8 | 0 |
| Example 31 | | MeCN [3] | | PGEE [4] | 6 | 80 | 8 | 9 |
| | | | | | 23 | 88 | 11 | 1 |
| Example 32 | | PGEE [3] | | MeCN [3.5] PGEE [1] | 6 | 96 | 3 | 0 |
| | | | | | 23 | 96 | 3 | 0 |
| Example 33 | | | | MeCN [2.5] PGEE [1] | 4 | 93 | 6 | 0 |
| | | | | | 23 | 93 | 6 | 0 |
| Example 34 | | PGEE [5] | | THF [3.5] | 6 | 96 | 3 | 0 |
| | | | | | 23 | 96 | 3 | 0 |
| Example 35 | | PGEE [4] | | | 6 | 95 | 4 | 0 |
| | | | | | 23 | 95 | 4 | 0 |
| Example 36 | | PGEE [3] | | | 6 | 94 | 5 | 1 |
| | | | | | 23 | 94 | 5 | 0 |
| Example 37 | | | | THF [2.5] PGEE [1] | 4 | 92 | 5 | 2 |
| | | | | | 23 | 94 | 5 | 0 |
| Example 38 | | | | MEK [2.5] PGEE [1] | 4 | 91 | 7 | 1 |
| | | | | | 23 | 92 | 7 | 0 |
| Example 39 | | | | DMF [2.5] PGEE [1] | 4 | 91 | 7 | 1 |
| | | | | | 23 | 92 | 7 | 0 |
| Example 40 | | | | DMAc [2.5] PGEE [1] | 4 | 90 | 8 | 1 |
| | | | | | 23 | 92 | 7 | 0 |

### [Example 41-1] Synthesis of arylsulfonic acid X-Cl-2

In accordance with the method described in WO 2009/096352 A, arylsulfonic acid X-2 represented by the following formula was synthesized, thereafter a sulfonyl halide compound (arylsulfonic acid X-Cl-2) was synthesized according to the following procedure.

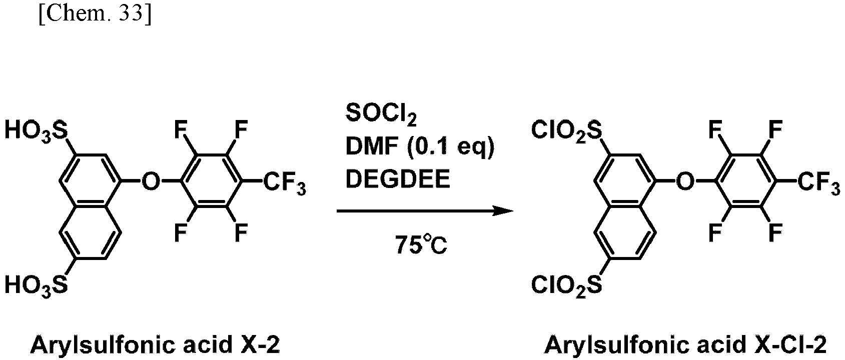

Under a nitrogen atmosphere, a solution obtained by dissolving 22.4 g of arylsulfonic acid X-2 in 20.2 g (0.9 times by weight) of DEGDEE at 40°C was added dropwise for 30 minutes to a mixed solution obtained by adding 33.8 g (1.5 times by weight) of thionyl chloride and 0.32 g (0.1 eq) of DMF to a 500 mL four-necked flask and stirring the resulting mixture at 75°C for 1 hour, then the used dropping funnel was washed with 11.37 g (0.5 times by weight) of DEGDEE and stirred at 75°C for 4 hours. At this time, acid gas is generated until the end of the reaction. Thus, the acid gas was released from the upper portion of the reflux tube to the outside of the system through the sodium hydroxide aqueous solution. In addition at this time, no lump was generated, and stirring was good. Furthermore, an arylsulfonic acid X-Cl-2 was precipitated during the dropwise addition. The reaction solution after 3 hours was subjected to HPLC analysis, and the result indicated that the reaction solution contained an arylsulfonic acid X-Cl-2 (98.0%) and a monosubstituted product (0.3%).

After confirming the termination of the reaction, 224 g (3 times by weight) of n-heptane was added dropwise at 73°C to 60°C over 1 hour. After the end of the dropwise addition, the resulting mixture was cooled to 25°C over 1 hour and stirred for 10 hours. The precipitated arylsulfonic acid X-Cl-2 was filtered under nitrogen pressure, and the filtered material was washed three times with 44.8 g (2 times by weight) of n-heptane. The obtained crystals were dried under reduced pressure at 50°C to give 20.5 g of arylsulfonic acid X-Cl-2 (yield: 85%, LC area: 98.4%, monosubstituted product 0.2%).

### [Example 41-2] Synthesis of arylsulfonic acid X-PGEE-2

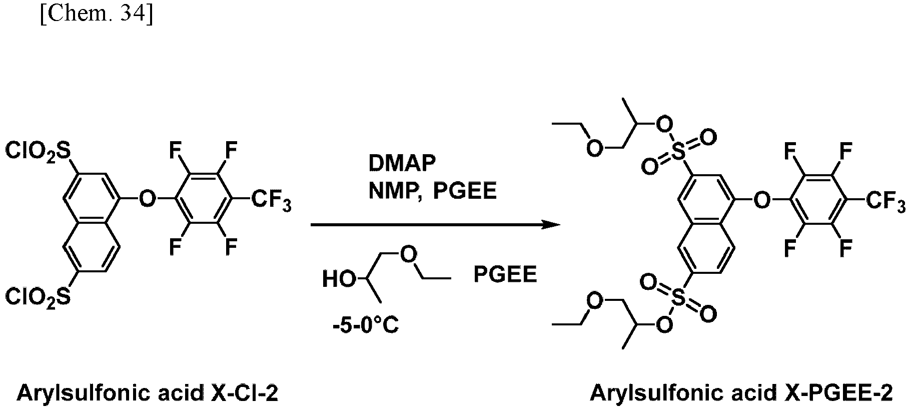

Under a nitrogen atmosphere, 2.5 g of arylsulfonic acid X-Cl-2 and 15 g (6 times by weight) of dehydrated PGEE were added to a 100 mL four-necked flask, and the resulting mixture was cooled to 2°C. After cooling, a mixed solution of 1.2 g (2.3 eq) of DMAP, 6.3 g (2.5 times by weight) of dehydrated NMP, and 2.5 g (1 time by weight) of dehydrated PGEE was added dropwise thereto at 2°C to 7°C over 11 minutes, and the resulting mixture was stirred at 2°C for 3 hours. The reaction solution after 3 hours was subjected to HPLC analysis, and the result indicated that the reaction solution contained arylsulfonic acid X-PGEE-2 (93.8%), a monosubstituted product (2.0%), and a raw material (0%). Thereafter, 23 g (9.2 times by weight) of ethyl acetate cooled to 4°C was added and stirred. At 5°C to 8°C, 12.53 g of 1M hydrochloric acid cooled to 5°C was added dropwise, and the mixture was continuously stirred at 5°C for 10 minutes, then allowed to stand for 5 minutes. After removing the aqueous layer, stirring was resumed, and 12.5 g (5 times by weight) of a 15 wt% ammonium chloride aqueous solution cooled to 5°C was again added dropwise at 5°C to 7°C, and the mixture was continuously stirred at 5°C for 10 minutes, then allowed to stand for 5 minutes. After removing the aqueous layer, stirring was resumed, and 12.6 g (5 times by weight) of a 15 wt% ammonium chloride aqueous solution cooled to 5°C was added dropwise at 5°C, and the mixture was continuously stirred at 5°C for 10 minutes, then allowed to stand for 5 minutes. After removing the aqueous layer, 11.25 g (4.5 times by weight) of diisopropyl ether was added. A 40 mm KIRIYAMA funnel was filled with 10 g (4 times by weight) of neutral silica gel 60N suspended in 30 g (12 times by weight) of a solution of ethyl acetate and diisopropyl ether with a weight ratio of ethyl acetate-to-diisopropyl ether of 2:1, and the separated solution was filtered. After the filtration, the mixture was further filtered with 47.5 g (19 times by weight) of a solution of ethyl acetate and diisopropyl ether with a weight ratio of ethyl acetate-to-diisopropyl ether of 2:1, and the silica gel on a filter was washed. The obtained solution was concentrated under reduced pressure to 7.7 g (3 times by weight) at 25°C, and isopropanol (25 g, 10 times by weight) was added dropwise thereto with stirring. After the dropwise addition, the resulting mixture was cooled to 1°C, and then stirred at 1°C for 2 hours. Thereafter, the precipitate was filtered, and the filtered material was washed twice with 2.5 g (1 time by weight) of diisopropyl ether. The obtained crystal was vacuum-dried at 25°C under reduced pressure for 3 hours to give 3.4 g of arylsulfonic acid X-PGEE-2 (yield: 78.9%, LC area: 98.0%) and a monosubstituted product (0.4%). The measurement results of ¹H-NMR and LC/MS are given below.

| | |
|---|---|
| ¹H-NMR (400MHz, CDCl₃): | δ 0.88-0.96 (m, 6H), 1.31, 1.33, 1.38, 1.40 (each s, 6H), |
| | 3,23-3,51 (m, 8H), 4.74-4.90 (m, 2H), 7.24 (d, J = 1.2Hz, 1H), |
| | 8.20 (d, J = 9.0, 1.8Hz, 1H), 8.43 (s, 1H), |
| | 8.55 (d, J = 9.0Hz, 1H), 8.67 (d, J = 1.8Hz, 1H) |
| LC/MS (ESI⁺) m/z | 693.1043 [M+H]⁺ |

## Claims

1. A method for producing an arylsulfonic acid ester compound, the method comprising:
a first step in which, when an arylsulfonic acid compound is reacted with a halogenation reagent in presence of a catalyst of N,N-dimethylformamide to produce an arylsulfonic acid halide, the arylsulfonic acid compound having formula (1) below: 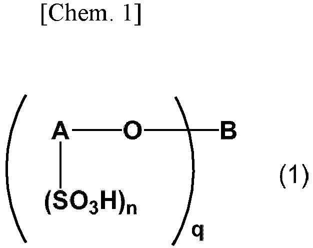 wherein A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
the arylsulfonic acid halide having formula (2) below: 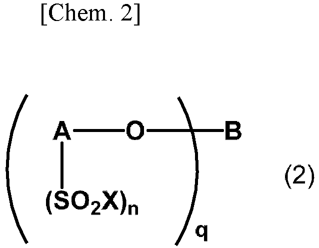 wherein X represents a halogen atom, and A, B, n and q each represent the same meaning as described above,
a solution of the arylsulfonic acid compound in 1,2-dimethoxyethane or in diethylene glycol diethyl ether is added dropwise into the halogenation reagent to halogenate the sulfo groups and synthesize the arylsulfonic acid halide; and
a second step of reacting the arylsulfonic acid halide obtained by the first step in an organic solvent and in presence of a base at 15°C or lower with an alcohol compound to synthesize the arylsulfonic acid ester compound, the base having formula (3) below: 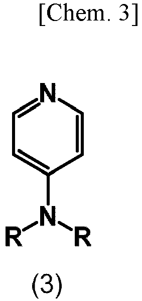 wherein Rs each independently represent a C1-C10 alkyl group optionally including a heteroatom, but two Rs may be bonded to form a ring structure together with a nitrogen atom,
the alcohol compound having formula (4) below: 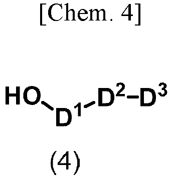 wherein D¹ represents a substituted or unsubstituted divalent hydrocarbon group, D² represents a single bond, O, S, or a substituted or unsubstituted divalent amino group, and D³ represents a substituted or unsubstituted monovalent hydrocarbon group, but D³ may be a hydrogen atom when D² is a single bond,
the arylsulfonic acid ester compound having formula (5) below: 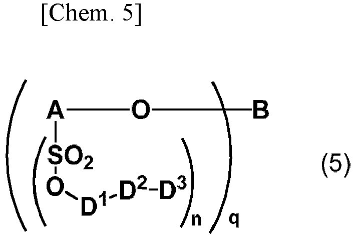 wherein A, B, D¹, D², D³, n and q each represent the same meaning as described above.

2. A method for producing an arylsulfonic acid ester compound, the method comprising:
reacting an arylsulfonic acid halide in an organic solvent and in presence of a base at 15°C or lower with an alcohol compound to synthesize the arylsulfonic acid ester compound, the arylsulfonic acid halide having formula (2) below: 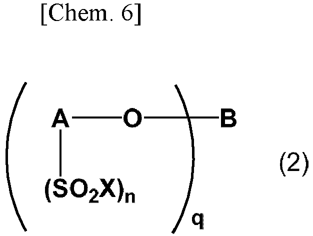 wherein X represents a halogen atom, A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
the base having formula (3) below: 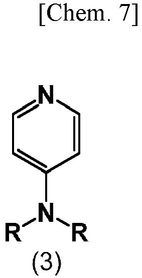 wherein Rs each independently represent a C1-C10 alkyl group optionally including a heteroatom, but two Rs may be bonded to form a ring structure together with a nitrogen atom,
the alcohol compound having formula (4) below: 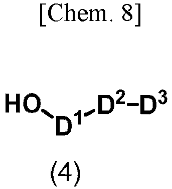 wherein D¹ represents a substituted or unsubstituted divalent hydrocarbon group, D² represents a single bond, O, S, or a substituted or unsubstituted divalent amino group, and D³ represents a substituted or unsubstituted monovalent hydrocarbon group, but D³ may be a hydrogen atom when D² is a single bond,
the arylsulfonic acid ester compound having formula (5) below: 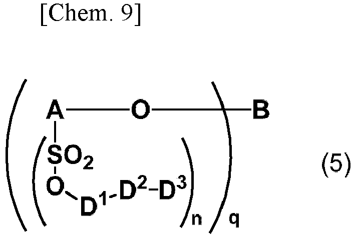 wherein A, B, D¹, D², D³, n and q represent the same meaning as described above.

3. The method for producing an arylsulfonic acid ester compound according to claim 1 or 2, wherein the base of the formula (3) is N,N-dimethyl-4-aminopyridine or 4-morpholinopyridine.

4. The method for producing an arylsulfonic acid ester compound according to any one of claims 1 to 3, wherein the reaction between the arylsulfonic acid halide and the alcohol compound is performed at -30 to 10°C.

5. The method for producing an arylsulfonic acid ester compound according to claim 4, wherein the reaction between the arylsulfonic acid halide and the alcohol compound is performed at -20 to 10°C.

6. The method for producing an arylsulfonic acid ester compound according to any one of claims 1 to 5, wherein the base of the formula (3) is used in an amount of 1.1 mol or more per 1 mol of sulfonic acid halide groups of the arylsulfonic acid halide.

7. The method for producing an arylsulfonic acid ester compound according to any one of claims 1 to 6, wherein the B is a C6-C20 q-valent aromatic group in which at least one hydrogen atom of the B is substituted with a fluorine atom.

8. The method for producing an arylsulfonic acid ester compound according to claim 7, wherein the B is a C6-C20 q-valent aromatic group in which all hydrogen atoms of the B are each substituted with a fluorine atom.

9. The method for producing an arylsulfonic acid ester compound according to any one of claims 1 to 8, wherein the q is 2.

10. The method for producing an arylsulfonic acid ester compound according to claim 9, wherein the B is a divalent group having formula (B1) below:

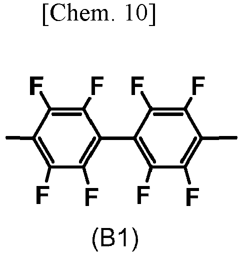

11. The method for producing an arylsulfonic acid ester compound according to any one of claims 1 to 8, wherein the q is 1.

12. The method for producing an arylsulfonic acid ester compound according to claim 11, wherein the B is a divalent group of any one having formulas (B2) to (B6) below:

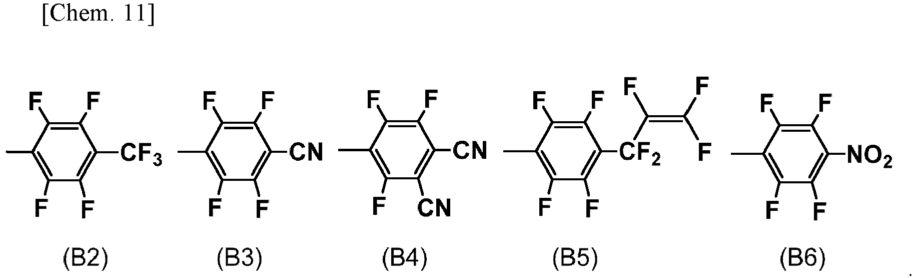

13. The method for producing an arylsulfonic acid ester compound according to any one of claims 1 to 12, wherein the A is a benzene ring or a naphthalene ring.

14. The method for producing an arylsulfonic acid ester compound according to claim 1 or any one of claims 3 to 13, wherein the halogenation reagent is thionyl chloride.

15. The method for producing an arylsulfonic acid ester compound according to claim 1 or any one of claims 3 to 14, wherein the first step further includes an operation of adding a poor solvent to a reaction solution that has been obtained to precipitate the arylsulfonic acid halide after synthesizing the arylsulfonic acid halide.

16. A method for producing an arylsulfonic acid halide by reacting an arylsulfonic acid compound with a halogenation reagent in presence of a catalyst of N,N-dimethylformamide, the arylsulfonic acid compound having formula (1) below: 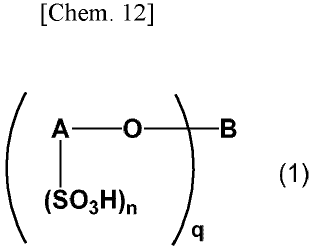 wherein A represents a C6-C20 (n+1)-valent aromatic group, B represents a C6-C20 q-valent aromatic group substituted with a halogen atom, n represents the number of sulfo groups bonded to the A and is an integer satisfying 1 ≤ n ≤ 4, and q represents the number of bonds between the B and an oxygen atom and is an integer satisfying 1 ≤ q,
the arylsulfonic acid halide having formula (2) below: 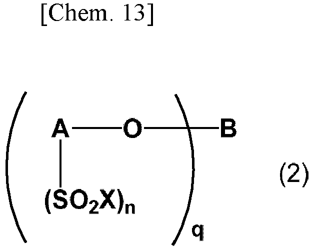 wherein X represents a halogen atom, and A, B, n and q represent the same meaning as described above,
the method comprising:
adding a solution of the arylsulfonic acid compound in 1,2-dimethoxyethane or in diethylene glycol diethyl ether dropwise into the halogenation reagent to halogenate the sulfo groups.

17. The method for producing an arylsulfonic acid halide according to claim 16, the method further comprising:
an operation of adding a poor solvent to a reaction solution that has been obtained to precipitate the arylsulfonic acid halide after synthesizing the arylsulfonic acid halide.
